(19) Europäisches Patentamt
European Patent Office
Office européen des brevets

(11) **EP 3 185 782 B1**

(12) **FASCICULE DE BREVET EUROPEEN**

(45) Date de publication et mention de la délivrance du brevet:
**02.06.2021 Bulletin 2021/22**

(21) Numéro de dépôt: **15771673.9**

(22) Date de dépôt: **28.08.2015**

(51) Int Cl.:
***A61B 10/00*** (2006.01)

(86) Numéro de dépôt international:
**PCT/FR2015/052292**

(87) Numéro de publication internationale:
**WO 2016/030642 (03.03.2016 Gazette 2016/09)**

(54) **DISPOSITIF D'OBTENTION DE MATIÈRE BIOLOGIQUE ET/OU D'INFORMATION BIOLOGIQUE A PARTIR D'UN ÉCHANTILLON A MATRICE HÉTÉROGENE**

VORRICHTUNG ZUR GEWINNUNG VON BIOLOGISCHEM MATERIAL UND/ODER BIOLOGISCHEN INFORMATIONEN AUS EINER PROBE MIT HETEROGENER MATRIX

DEVICE FOR OBTAINING BIOLOGICAL MATERIAL AND/OR BIOLOGICAL INFORMATION FROM A SAMPLE WITH A HETEROGENEOUS MATRIX

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.08.2014 FR 1458143**

(43) Date de publication de la demande:
**05.07.2017 Bulletin 2017/27**

(73) Titulaire: **Biomérieux**
**69280 Marcy-L'Etoile (FR)**

(72) Inventeurs:
• ROSTAING, Hervé
**F-38420 Le Versoud (FR)**
• DUPONT-FILLIARD, Agnès
**F-38190 Les Adrets (FR)**
• GICQUEL, Sandrine
**F-38100 Grenoble (FR)**
• VACHON, Carole
**F-38960 St Etienne de Crossey (FR)**
• BROYER, Patrick
**F-38500 Saint Cassien (FR)**
• BLAZE, Jérôme
**38660 La Terrasse (FR)**

(74) Mandataire: **Schoen, Elsa Imilia**
**Biomérieux SA**
**376, chemin de l'Orme**
**69280 Marcy-l'Étoile (FR)**

(56) Documents cités:
EP-A1- 1 656 887     WO-A1-03/014705
US-A- 4 081 356      US-A- 4 735 905
US-A1- 2005 155 440  US-A1- 2006 115 385
US-A1- 2011 048 981

EP 3 185 782 B1

**Description**

**Domaine technique**

La présente invention concerne le domaine de l'obtention de matière biologique et/ou d'information biologique à partir d'un échantillon à matrice hétérogène aux fins d'analyses ultérieures, en particulier à des fins de diagnostic.

**Etat de la technique**

Le contrôle de matière biologique et/ou d'information biologique à partir d'échantillons d'origines diverses (par exemple d'origine industrielle, agro-alimentaire ou clinique) requiert la mise en œuvre de techniques qui permettent la détection - par exemple aux fins d'identification et/ou de dénombrement de micro-organismes - et dont le rendu en termes de résultats doit être le plus efficace possible. D'une manière générale, si l'on considère ladite matière biologique, cette dernière peut être de nature non pathogène. Toutefois, dans le domaine du diagnostic, la matière biologique faisant l'objet d'un contrôle est le plus fréquemment pathogène, nécessitant dès lors une détection rapide et précise de celle-ci afin d'engager les actions correctives *ad hoc* le plus tôt possible. Bien évidemment, les toxines ou autres métabolites produites par cette matière biologique pathogène peuvent également être recherchées.

De façon standard, la détection de matière biologique et/ou d'information biologique peut être effectuée par des essais immunologiques, des essais enzymatiques, des techniques de biologie moléculaire, ou encore, pour ce qui concerne ladite matière biologique, par dénombrement et/ou identification sur boîte de culture. En tout état de cause, qu'il s'agisse de matière biologique et/ou d'information biologique, la sensibilité de détection et la spécificité de détection sont deux facteurs importants pour évaluer l'efficacité du test effectué.

A titre d'exemple, la détection des micro-organismes est, de manière conventionnelle, effectuée sur des milieux de culture sélectifs étalés sur boîte de Petri, comme recommandé par les normes de type ISO (« International Organization for Standardization », en langue anglaise) ou les méthodes BAM-FDA (« Bacteriological Analytical Manual of the Food and Drug Administration », en langue anglaise). Ces normes recommandent notamment l'emploi de milieux tels que le « polymyxin egg yolk mannitol bromothymol blue Agar » (dont l'acronyme est PEMBA) ou le « mannitol-egg yolk-polymyxin Agar » (dont l'acronyme est MYP), selon leurs dénominations en langue anglaise. L'identification de micro-organismes est effectuée, de manière classique, selon des critères morphologiques, culturaux et/ou métaboliques. Néanmoins, ces méthodes de détection sur boîte de Petri peuvent être impactées, de manière négative, par la présence d'éléments non-spécifiques, issus de l'échantillon à analyser. Ceci est particulièrement vrai en ce qui concerne les échantillons à matrice hétérogène, à savoir présentant des différences en termes de consistance (parties solides, parties plus ou moins liquides, parties semi-solides etc.), tels que des échantillons de sol, selles humaines ou animales, glaires, crachats ou encore des échantillons de tissus (échantillons médico-légaux), lesquels sont généralement riches en polysaccharides, débris cellulaires, et inhibiteurs de toutes sortes (sel biliaire, polyphénols, polysaccharide, fibres, hémoglobine,...), susceptibles de perturber la croissance des micro-organismes recherchés et donc de donner lieu à des résultats faussement négatifs (problème de sensibilité de détection).

Alternativement, la détection d'information biologique issue de la matière biologique peut être réalisée, par exemple, par des méthodes d'amplification d'acides nucléiques classiques, spécifiques d'un ou plusieurs types de micro-organismes et de virus recherchés. Toutefois ces techniques sont sensibles et susceptibles d'être inhibées de manière importante par des composés tels que des polysaccharides, des débris cellulaires et inhibiteurs de toutes sortes. Cela peut conduire à l'obtention de résultats faussement négatifs (problèmes de sensibilité de détection) ou à un diagnostic inexact et résulter en de graves conséquences pour le patient humain ou animal. Par conséquent, il est primordial d'isoler les micro-organismes et/ou les virus de sorte qu'un minimum d'inhibiteurs soient présents dans l'isolat. Ceci est particulièrement important pour les échantillons à matrice hétérogène, tels que mentionnés précédemment. En effet, ceux-ci comprennent un grand nombre de constituants non-spécifiques (polysaccharides, débris cellulaires, inhibiteurs de toutes sortes), susceptibles de générer des faux-négatifs lors de la mise en œuvre des méthodes d'amplification d'acides nucléiques susvisées.

En outre, et quelle que soit le type de méthode de détection utilisée, ces échantillons à matrice hétérogène présentent, en sus des problématiques classiques de contamination de l'échantillon et/ou de l'environnement, des problématiques plus spécifiques de dosage/calibration (corrélation difficile entre quantité de l'échantillon prélevé et masse de celui-ci, moyen de calibration constant d'un échantillon à l'autre) et de mise en suspension dans un milieu liquide, inhérentes à la nature de l'échantillon.

Actuellement, il existe différentes méthodes permettant d'analyser le matériel génétique contenu dans un échantillon à matrice hétérogène. A titre d'exemple, le kit « QIAamp® DNA » de la société QiaGen permet d'isoler et purifier l'ADN génomique total d'un échantillon solide ou liquide (tel que le sang, le sérum, le plasma, etc.). Ce kit met en œuvre un dispositif consistant en des tubes et des colonnes de filtration adaptés pour une utilisation avec des micro-centrifugeuses standard. Selon les recommandations du fournisseur, la méthode pour obtenir l'ADN génomique total comprend les étapes suivantes : prélever une quantité déterminée de l'échantillon d'intérêt (par pesée), en fonction de sa nature, introduire celui-ci dans un tube contenant une solution pour sa mise en suspension, mettre l'échantillon en suspension par une agitation vigoureuse de type vortex. Une fois l'échantillon malaxé vigoureusement, une solution de lyse est ajoutée et une étape d'incubation est réalisée pendant plusieurs minutes, afin de rompre les membranes cellulaires et de libérer l'information génétique dans la solution. Ensuite, afin d'éliminer les débris, la solution est centrifugée et le surnageant prélevé. Il s'ensuit plusieurs étapes laborieuses d'additions de solutions et de centrifugations permettant de purifier l'échantillon d'ADN, avant de l'éluer sur colonne par centrifugation.

Cependant cette méthode, mettant en œuvre le kit « QIAamp® DNA » susvisé, présente plusieurs inconvénients, notamment lorsque l'échantillon testé est un échantillon à matrice hétérogène. En effet, les premières étapes de pesée et de transfert de l'échantillon dans les tubes de micro centrifuge sont critiques en raison de l'étroitesse de l'ouverture desdits tubes. Ce transfert peut donc s'avérer délicat et nécessiter des manipulations et des équipements supplémentaires, et des temps de réalisation allongés. En outre, lors de ces opérations de pesée et/ou de transfert, il existe un risque significatif de :

- perte de la matière,
- contamination de l'échantillon et/ou de l'environnement et/ou du technicien de laboratoire,
- l'inconfort du technicien de laboratoire lié à la manipulation répété d'échantillons de type selles, crachats ou glaires,
- la difficulté de la calibration de l'échantillon par pesée.

Par ailleurs, les nombreuses étapes constituant le protocole du kit « QIAamp® DNA », qui impliquent la manipulation d'une quantité importante de tubes différents, sont susceptibles d'engendrer des erreurs de manipulation. De surcroît, la manipulation de tubes de petite taille, tels que des tubes adaptés aux micro-centrifugeuses, s'avère peu pratique pour le technicien de laboratoire, ce qui est également susceptible de donner lieu à des pertes de temps et d'efficacité. D'autre part, le kit « QIAamp® DNA » permet uniquement l'isolation d'acide nucléique d'un échantillon, mais ne permet pas de détecter la présence de micro-organismes viables, comme par exemple une détection sur milieu de culture. De plus, le kit « QIAamp® DNA » ne permet pas la détection de levure à partir d'un échantillon de selles.

Dans un autre exemple, le brevet US 4,081,356 décrit un dispositif et une méthode permettant de récupérer de petits sédiments contenant des œufs de parasites et des larves à partir d'un échantillon de matière fécale. Le dispositif divulgué consiste en deux compartiments séparés par un élément de filtration sommaire (à savoir très peu sélectif), tel qu'un filtre et une maille en acier ; le compartiment supérieur étant obstrué à son extrémité externe par une spatule permettant la prise d'échantillon. L'échantillon de matière fécale est prélevé à l'aide de la spatule, laquelle est adaptée pour être connectée sur la partie supérieure du compartiment. Ensuite, l'échantillon est émulsifié en utilisant une solution de formaldéhyde, éventuellement supplémentée avec un agent tensioactif, de manière mécanique à l'aide d'un bâtonnet. L'émulsification est finalisée par une étape d'agitation horizontale en présence de billes de verre. Puis une étape d'agitation verticale permet de faire passer la totalité de l'échantillon émulsifié dans le compartiment inférieur du dispositif, au travers de l'élément de filtration sommaire. De l'éther est ensuite utilisé pour rincer cet élément de filtration sommaire et permettre la formation d'un mélange au niveau du compartiment inférieur. Enfin, le compartiment inférieur est agité et centrifugé pour séparer le mélange ainsi obtenu en différentes couches de matières de densités différentes, afin d'isoler la couche de sédiment contenant les œufs de parasites et les larves.

Dans un autre exemple, le brevet US 7,648,681 décrit un dispositif selon le préambule de la revendication 1.

Cependant, le dispositif utilisé dans la méthode susvisée présente plusieurs inconvénients, notamment liés au manque de sélectivité flagrant de l'élément de filtration sommaire, lequel autorise le passage, outre des œufs et des larves susvisés, d'éléments non-spécifiques qui vont ensuite sédimenter en différentes couches. Par ailleurs, les premières étapes de prélèvement d'échantillon, en particulier l'étape de connexion de la spatule avec le compartiment supérieur, ne permettent pas un prélèvement d'échantillon propre et aisé, et nécessitent, en conséquence, des manipulations supplémentaires et des temps de réalisation allongés. De surcroît, ceci affecte inévitablement la reproductibilité de la méthode mettant en œuvre ce dispositif, dans la mesure où il s'avère extrêmement délicat - voire impossible - de prélever un volume précis et calibré dudit échantillon, notamment sur des selles dures de type Bristol 1 et 2. Les selles humaines sont communément caractérisées et classées selon l'échelle de Bristol consistant en une classification visuelle répartissant les selles humaines en sept types. Les types 1-2 correspondent à des selles dures, les types 3-4 sont considérés comme normaux, et les types 5-6-7 correspondent à des selles de consistance liquide.

Eu égard aux problématiques présentées ci-dessus, un objectif de la présente invention vise à mettre au point un dispositif et son/ses procédé(s) associé(s) permettant :

- de doser/calibrer la quantité d'échantillon à matrice hétérogène prélevée sans avoir besoin de peser ce dernier,
- de mettre en suspension efficacement ledit échantillon dans un milieu liquide,
- de limiter les problèmes liés au colmatage (encombrement) des filtres pour faciliter la filtration et améliorer la récupération de la matière biologique et/ou de l'information biologique,

- de limiter l'obtention d'éléments non-spécifiques susceptibles de fausser le résultat de l'analyse *in fine*,
- d'éviter la perte d'une partie de l'échantillon lors du transfert d'échantillon dans le contenant (par exemple dans le tube),
- d'améliorer l'efficacité et la praticité,
- de limiter au maximum les risques de contamination de l'échantillon et/ou de l'environnement et/ou du technicien de laboratoire,
- de simplifier le protocole de préparation d'échantillons à matrice hétérogène afin de réduire le temps opérateur, le nombre de matériels et de consommables utilisés, les risques d'erreurs et améliorer la reproductibilité, de proposer un dispositif compatible avec l'ensemble des méthodes d'analyse connues (microbiologie, culture, virologie, bactériologie, immunoassay, métaséquençage, PCR...),
- d'isoler les levures.

D'autres objectifs apparaîtront, le cas échéant, à la lecture de la présente demande de brevet.

**Exposé de l'invention**

En conséquence, l'objet de la présente invention concerne un dispositif d'obtention de matière biologique et/ou d'information biologique selon la revendication 1.

Ainsi, le dispositif selon l'invention permet de simplifier et faciliter le prélèvement d'une masse déterminée d'un échantillon à matrice hétérogène (notamment en s'affranchissant d'une délicate étape de pesée), sa mise en suspension et l'isolement de la matière biologique et/ou de l'information biologique qu'il contient, en vue de son analyse ultérieure. En particulier, le dispositif selon l'invention présente l'avantage d'être adapté pour traiter des échantillons à matrice hétérogène de consistance et/ou de texture très variée(s).

La simplification de l'isolement de la matière biologique et/ou de l'information biologique obtenue grâce au dispositif selon l'invention entraîne notamment une diminution des étapes à « tube ouvert », lesquelles nécessitent des consommables et des manipulations techniques (pipetage, retrait de surnageant, centrifugation...), susceptibles d'engendrer des erreurs et des contaminations de l'échantillon et/ou du milieu extérieur. Le dispositif selon l'invention permet également de s'affranchir de l'utilisation d'une multitude d'équipements, tels que des balances, des broyeurs à billes (« bead beater » en langue anglaise) ou des centrifugeuses.

Par « échantillon à matrice hétérogène », l'on entend une petite partie ou une petite quantité isolée d'une matière à matrice hétérogène, pour l'analyse. La matière à matrice hétérogène à partir de laquelle l'échantillon est prélevé est caractérisée par des différences intrinsèques en termes de consistance et/ou de texture (parties solides, parties plus ou moins liquides, parties semi-solides, parties visqueuses/viscoélastiques). Cette matière à matrice hétérogène est susceptible de contenir la matière biologique et/ou l'information biologique recherchée(s). A titre d'exemple, l'échantillon à matrice hétérogène peut être un échantillon de sol, de selles humaines ou animales, de glaires, de crachats, un échantillon industriel ou encore un échantillon de tissus (échantillons médico-légaux). De préférence, l'échantillon à matrice hétérogène est un échantillon de selles humaines ou animales. Selon un mode de réalisation particulièrement préféré, cet échantillon à matrice hétérogène est un échantillon de selles humaines. Le dispositif selon la présente invention permet l'obtention de matière biologique et/ou d'information biologique à partir de l'ensemble des types de selles définis par l'échelle de Bristol, à savoir les types 1-7.

Par « matière biologique », l'on entend, au sens de la présente invention toute matière contenant des informations génétiques et qui est autoreproductible ou reproductible dans un système biologique. A titre d'exemple de matière biologique autoreproductible, l'on peut citer une « matière biologique » autoreproductible microscopique, telle qu'une ou plusieurs cellule(s) humaine(s), animale(s) ou végétale(s) ou une/plusieurs bactéries/levures.

Selon un mode de réalisation préféré de l'invention, ladite matière biologique est une matière microbiologique autoreproductible (telle qu'une ou plusieurs bactérie(s), levure(s), champignon(s)) ou reproductible (telle qu'un ou plusieurs virus) dans un système biologique. Le dispositif selon la présente invention est particulièrement bien adapté pour détecter et/ou identifier et/ou dénombrer des micro-organismes pathogènes pour l'homme et/ou l'animal.

Par « information biologique », l'on entend, au sens de la présente invention, tout élément constituant ladite matière biologique ou produit par cette dernière, tel que des acides nucléiques (ADN, ARN), des protéines, des peptides ou des métabolites. L'information biologique peut notamment être contenue au sein de ladite matière biologique ou excrétée/sécrétée par cette dernière.

Ladite « partie creuse calibrée», de préférence de forme concave, permet de délimiter un volume prédéterminé, adapté pour prélever une masse prédéfinie de la matière à matrice hétérogène. Plus précisément, le volume de cette partie creuse est déterminé pour contenir une quantité de matière biologique et/ou d'information biologique suffisante pour permettre son/leur analyse.

Selon un mode de réalisation préféré, le moyen de prélèvement calibré comprend une tige et une partie creuse calibrée.

Selon ce mode de réalisation préféré, ladite partie creuse calibrée est connectée à la partie interne du bouchon par l'intermédiaire de ladite tige.

Avantageusement, afin d'éviter de prélever une masse/quantité excédentaire de matière biologique et/ou d'information biologique, ladite tige est adaptée pour que seule ladite au moins une partie creuse calibrée soit susceptible de contenir de l'échantillon à matrice hétérogène. A cet effet, ladite tige est, de préférence, dépourvue de tout renforcement ou cavité, évidemment à l'exception de ladite partie creuse calibré. Selon un mode de réalisation particulier, ladite tige est en matériau plein.

Selon un mode de réalisation préféré, ladite partie creuse calibrée est une partie évidée de ladite tige, avantageusement située au niveau de l'extrémité de la tige opposée à la partie interne du bouchon.

Selon un mode de réalisation préféré, ladite partie creuse possède au moins une paroi adaptée pour enlever le surplus d'échantillon à matrice hétérogène en « raclant » ladite partie creuse calibrée contre une surface appropriée, de constitution suffisamment rigide. Ainsi, en enlevant le surplus d'échantillon à matrice hétérogène, faisant saillie à l'extérieur du volume défini par la partie creuse calibrée, l'opérateur s'assure que la masse d'échantillon à matrice hétérogène prélevée n'excède pas, de manière significative, la masse souhaitée, correspondant au volume de la partie creuse calibrée.

De manière avantageuse, ladite partie creuse est calibrée pour contenir une masse comprise entre 10 mg et 2000 mg, de préférence entre 100 mg et 1000 mg, avantageusement entre 200 mg et 300 mg, de ladite matière à matrice hétérogène.

Selon un mode de réalisation particulier de l'invention, ladite tige comprend - ou est connectée à - au moins une partie coulissante, permettant de passer d'une première longueur de tige vers une deuxième longueur de tige (et, de préférence, inversement, à savoir de passer de ladite deuxième longueur de tige vers ladite première longueur de tige), ladite deuxième longueur de tige étant inférieure à ladite première longueur de tige.

Selon un premier aspect de ce mode de réalisation particulier, ladite tige peut être - au moins partiellement - télescopique.

Selon un deuxième aspect de ce mode de réalisation particulier, particulièrement avantageux, la susdite partie coulissante est une extension coulissante, adaptée pour être positionnée sur ladite tige et coulisser le long de cette dernière afin de passer de ladite première longueur de tige vers ladite deuxième longueur de tige (et, de préférence, inversement), ladite extension coulissante comprenant au moins une partie creuse calibrée, de préférence positionnée au niveau de l'extrémité de l'extension coulissante la plus éloignée de la partie interne du bouchon.

Selon un mode de réalisation particulier de l'invention, le moyen de prélèvement est adapté pour contenir plusieurs parties creuses calibrées, de préférence de 2 à 10, préférentiellement de 2 à 6, avantageusement 3 ou 6, pouvant contenir chacune entre 10 mg et 2000 mg de ladite matière à matrice hétérogène, de préférence entre 100 mg et 1000 mg, avantageusement entre 200 mg et 300 mg, de ladite matière à matrice hétérogène. En conséquence, selon ce mode de réalisation particulier, le moyen de prélèvement selon l'invention peut être adapté pour prélever une quantité d'échantillon à matrice hétérogène comprise entre 20 mg et 20000 mg, préférentiellement entre 200 mg et 6000 mg. De manière évidente, l'homme du métier saura adapter le moyen de prélèvement selon l'invention, notamment en ce qui concerne le nombre et/ou le volume de la/des partie(s) creuse(s) calibrée(s), notamment afin d'obtenir *in fine* une concentration en matière biologique et/ou information biologique compatible avec le niveau de sensibilité minimum des techniques d'analyse. A titre d'exemple, il est bien connu de l'homme du métier que les micro-organismes responsables de la tuberculose bovine (mycobactéries du complexe tuberculosis) sont difficilement détectables et nécessitent, en conséquence, une quantité plus importante d'échantillon afin d'obtenir une concentration en micro-organismes analysable.

Dans un mode de réalisation particulier, ledit moyen de prélèvement présente, de chaque côté de la tige, 3 parties creuses calibrées pouvant contenir chacune un volume correspondant à 1000 mg, soit un volume total correspondant à 6000 mg, d'échantillon à matrice hétérogène, tel que des fèces bovins, ovins, caprins ou porcins, avantageusement de bovins.

Typiquement, l'analyse de selles d'origine humaine est réalisée à partir de 200 à 300 mg d'échantillon. Toutefois, dans certains cas particuliers, cela peut aller jusqu'à 1000 mg. Par ailleurs, dans le cas d'applications vétérinaires, la masse d'échantillon nécessaire aux fins de l'analyse peut représenter plusieurs grammes, voire plusieurs dizaines de grammes.

Selon un mode de réalisation préféré de l'invention, l'assemblage du bouchon et du contenant s'opère par coopération étroite des deux entités via un système de fermeture, tel qu'un système de vissage ou de clipsage, permettant de fermer ledit contenant de sorte que le contenu puisse uniquement sortir sous forme de filtrat par ladite au moins une ouverture dudit bouchon lorsque ledit contenant est fermé par ledit bouchon.

Par « moyen d'obturation amovible », l'on entend, au sens de la présente invention tout moyen permettant d'empêcher toute sortie non contrôlée de filtrat. A titre d'exemple, ledit moyen d'obturation peut être un capuchon ou une partie sécable de protection. Dans ce dernier cas, ladite partie sécable de protection est solidaire de ladite ouverture, de préférence est scellée à cette dernière (par exemple par thermosoudage) et s'avère sécable, par exemple, par torsion ou traction. Alternativement, cette partie sécable de protection peut être coupée sous l'action d'un moyen tranchant, tel qu'un couteau ou une paire de ciseaux. Il est intéressant de noter que ladite partie sécable remplit également la fonction de témoin d'inviolabilité, dans la mesure où le fait que cette partie sécable se trouve désolidarisée du dispositif signifie, pour l'opérateur, que le dispositif en question a déjà été utilisé. En d'autres termes, cette partie sécable permet d'assurer l'intégrité du dispositif selon l'invention.

De manière avantageuse, lorsque le moyen d'obturation amovible selon l'invention est une partie sécable de protection, cette dernière possède sur l'extrémité opposée à ladite ouverture, une forme complémentaire à ladite ouverture. Ainsi lorsque ladite partie sécable de protection est désolidarisée de ladite ouverture, par exemple en appliquant une force de traction ou de torsion sur ladite partie sécable, l'extrémité de cette partie sécable possédant une forme complémentaire à ladite ouverture peut être positionnée sur cette dernière afin de l'obturer. Dans ce mode de réalisation préféré, ladite partie sécable de protection, une fois désolidarisée de l'ouverture, joue le rôle de capuchon ; ladite partie sécable de protection pouvant donc être qualifiée de « capuchon sécable de protection ».

Tel qu'indiqué précédemment, le moyen de prélèvement calibré selon l'invention est connecté à la partie interne du bouchon par tout moyen approprié. Ainsi, ledit moyen de prélèvement calibré peut être vissé, collé, assemblé par emboîtage élastique (par exemple par clipsage) ou encore enfiché en force dans une ouverture pratiquée dans ledit bouchon. Selon un mode de réalisation particulier, ce moyen de prélèvement calibré peut être thermosoudé à la partie interne du bouchon.

Selon l'invention, ledit contenant comprend en outre au moins un moyen de mise en suspension mécanique, de préférence de forme sphérique telle qu'une bille, adapté pour faciliter la mise en suspension dudit échantillon dans ladite solution de mise en suspension.

Par « moyen de mise en suspension mécanique », l'on entend un élément adapté pour être disposé au sein du contenant selon l'invention et pour être mis en mouvement par un ou plusieurs système(s) de force(s) externe(s) au dispositif selon l'invention, par exemple par une agitation manuelle ou induite par l'appareil de malaxage de type vortex, sonificateur ou agitateur magnétique, broyeur à billes (« bead beater » en langue anglaise) afin de permettre/faciliter la mise en suspension mécanique de l'échantillon au sein de la dite solution de mise en suspension.

Avantageusement, le dispositif comprend un nombre de moyens de mise en suspension mécanique, tels que des billes, sélectionné entre 1 et 200, de préférence entre 5 et 50, avantageusement entre 10 et 40, de manière particulièrement préférée entre 25 et 35; ledit/lesdits moyens de mise en suspension mécanique ayant une taille comprise entre 1 mm et 10 mm, de préférence entre 2,5 mm et 3,5 mm, avantageusement d'environ 3 mm ; de préférence, ledit/lesdits moyens de mise en suspension mécanique étant en verre, fer, plastique(s), céramique(s), de manière particulièrement préférée en verre.

La taille du ou des moyen(s) de mise en suspension mécanique s'entend de la plus grande dimension de ce(s) dernier(s). Par exemple, la taille d'un moyen de mise en suspension mécanique ayant la forme d'un parallélépipède rectangle (pavé droit) s'entend de la longueur (dimension la plus grande) de ce parallélépipède rectangle.

Tel qu'indiqué précédemment, ledit au moins un moyen de mise en suspension mécanique est de forme sphérique et consiste, avantageusement, en une bille. Dans le cas d'un moyen de mise en suspension mécanique de forme sphérique, la taille s'entend du diamètre de celui-ci. Ainsi, lorsque l'on utilise, en tant que moyen de mise en suspension mécanique, une pluralité de billes ayant une taille comprise entre 2 mm et 10 mm, de préférence entre 2,5 mm et 3,5 mm, avantageusement d'environ 3 mm, il convient de comprendre que le diamètre des billes est compris entre 2 mm 10 mm, de préférence entre 2,5 mm et 3,5 mm, avantageusement lesdites billes ont un diamètre d'environ 3 mm. Préférentiellement, ladite taille desdites billes est en accord avec la forme et les dimensions de ladite partie creuse calibrée.

De préférence, la forme et/ou la taille dudit au moins un moyen de mise en suspension mécanique est/sont adaptée(s) pour ne pas obturer ladite au moins une ouverture permettant la communication de fluide entre l'intérieur dudit contenant et l'extérieur dudit dispositif lorsque ledit bouchon et ledit contenant sont assemblés. A l'inverse, lorsque l'on connaît, par avance, la taille et/ou la forme dudit au moins un moyen de mise en suspension mécanique, la forme et/ou la taille de ladite ouverture peut(vent) être adaptée(s) en conséquence, afin d'atteindre le même but.

Selon l'invention, le(s) moyen(s) de mise en suspension mécanique a/ont une taille (un diamètre dans le cadre de moyen(s) de mise en suspension mécanique de forme sphérique) adaptée pour coopérer avec ladite au moins une partie creuse du moyen de prélèvement calibré, notamment afin de faciliter la mise en suspension de l'échantillon à matrice hétérogène prélevé au préalable.

Préférentiellement, le(s) moyen(s) de mise en suspension mécanique est/sont constitué(s) d'un matériau adapté pour interagir avec un système/dispositif externe de mise en suspension, ledit système/dispositif permettant de mettre en mouvement ou en vibration le(s) moyen(s) de mise en suspension mécanique (de préférence les billes). Un tel système/dispositif peut consister, par exemple, en un sonificateur (également dénommé « sonicateur ») ou un agitateur magnétique. De manière optimale, le matériau dont est/sont constitué(s) le(s) moyen(s) de mise en suspension mécanique interagit peu ou pas avec ladite matière biologique et/ou information biologique, notamment afin de ne pas altérer/dégrader ladite matière biologique et/ou ladite information biologique.

Avantageusement, afin de faciliter la manipulation, ledit moyen de prélèvement calibré possède une rigidité suffisante pour éviter que ledit moyen de prélèvement calibré ne se courbe lors du prélèvement d'échantillon et pour garantir un faible coefficient de variation sur la masse de matrice biologique prélevée.

Par « rigidité suffisante », l'on entend une rigidité adaptée pour effectuer des prélèvements sur tous types d'échantillon à matrice hétérogène, et notamment ceux présentant une consistance relativement solide. La rigidité suffisante - et, par conséquent, la faible élasticité - du moyen de prélèvement calibré permet de faciliter le prélèvement et d'éviter les projections d'échantillon à matrice hétérogène lors de l'opération de prélèvement.

Avantageusement, la rigidité suffisante du moyen de prélèvement est obtenue par l'emploi de matériaux permettant d'atteindre le degré de rigidité requis, tels que le polypropylène (PP), résine Delrin, matériaux thermoplastiques polyamides (PA), polycarbonate (PC), polyméthacrylate de méthyl (PMMA), Polyéthylène basse densité (PEBD), acrylnitrile-butadiene-styrene (ABS) et/ou par une forme favorisant la rigidité du moyen de prélèvement. En outre, afin d'éviter que le moyen de prélèvement calibré selon l'invention ne se courbe lors du prélèvement d'échantillon, il s'avère important que la connexion entre ledit moyen de prélèvement et la partie interne du bouchon soit suffisamment rigide afin d'éviter qu'elle ne plie/se courbe lors de l'opération de prélèvement d'échantillon. En effet, lors de cette opération, l'opérateur tient généralement entre au moins deux de ses doigts la partie externe du bouchon puis effectue le prélèvement désiré. Il est donc important qu'il n'existe aucun point faible au niveau de la connexion entre partie interne du bouchon, d'une part, et moyen de prélèvement calibré, d'autre part.

Selon un mode de réalisation particulier, ladite partie creuse calibrée comporte au moins une ouverture permettant de faciliter la mise en suspension dudit échantillon dans ladite solution de mise en suspension.

De préférence, ladite au moins une ouverture possède une forme circulaire, ovoïde ou allongée (préférablement oblongue). De préférence, ladite ouverture est de forme allongée, avantageusement oblongue, présentant, par exemple, une forme en bâtonnet. Cette forme allongée - et de préférence oblongue - permet d'assurer un prélèvement calibré et reproductible de l'échantillon à matrice hétérogène tout en favorisant la suspension de celui-ci dans la solution de mise en suspension. Selon un mode de réalisation particulièrement préféré, ladite au moins une ouverture possède une forme et/ou une taille adaptée(s) pour coopérer avec au moins un moyen de mise en suspension mécanique tel que défini précédemment, afin de favoriser la mise en suspension de l'échantillon à matrice hétérogène prélevé par l'opérateur.

Selon un mode de réalisation particulier, afin de faciliter d'avantage cette mise en suspension, ladite au moins une partie creuse est revêtue sur tout ou partie de sa surface (préférablement sur l'intégralité de sa surface) d'un revêtement antiadhésif, tel qu'une ou plusieurs couche(s) de matière(s) hydrosoluble(s) ou encore un revêtement de type hydrophobe. De préférence, et afin de faciliter la fabrication du dispositif, le matériau choisi pour la réalisation de la partie creuse calibrée est naturellement à tendance hydrophobe et/ou présente une faible rugosité. A titre d'exemple, cette faible rugosité peut notamment être obtenue par polissage pour donner une finition du type poli-miroir.

Selon un mode de réalisation préféré, ledit contenant comprend ladite solution de mise en suspension, par exemple une solution tampon, dans un volume suffisant pour permettre la mise en suspension de l'échantillon.

Cette mise en suspension peut être réalisé :

- de manière directe, à savoir en immergeant partiellement ou totalement - de préférence totalement - ladite au moins une partie creuse calibrée contenant l'échantillon dans la solution de mise en suspension, ou
- de manière indirecte, en induisant une variation de niveau de la solution de mise en suspension, de sorte que cette dernière entre en contact par intermittence (à intervalles réguliers ou irréguliers) avec tout ou partie de ladite au moins une partie creuse calibrée contenant l'échantillon.

La mise en suspension « indirecte » de l'échantillon à matrice hétérogène dans la solution de mise en suspension peut notamment se produire lors d'une étape de mise en suspension mécanique, à savoir, par exemple, lors d'une étape d'agitation/ de malaxage. Cette dernière peut être obtenue, par exemple, en utilisant un agitateur ou un appareil de malaxage de type vortex.

A titre d'exemple de « solution de mise en suspension », peut être utilisée toute solution adaptée pour :

i) permettre la mise en suspension dudit « échantillon à matrice hétérogène », et/ou
ii) préserver la matière biologique et/ou l'information biologique d'intérêt (à savoir éviter toute dégradation de cette dernière).

Ainsi, ladite solution de mise en suspension peut être un tampon phosphate (mono/dibasique, EDTA 10 mM, pH 8), un tampon PBS ou un tampon TE (« Tris-EDTA » ; 10 mM Tris, 10 mM EDTA (Acide Ethylène Diamine Tétra acétique), pH 8). De manière intéressante, ladite solution de mise en suspension peut également comprendre un élément conservateur préservant la faune microbienne de l'échantillon prélevé, à savoir évitant toute modification de celle-ci, afin de permettre le transport de l'échantillon à température ambiante du lieu de prélèvement au lieu d'analyse, ou bien de différer l'étape d'analyse. Un conservateur de ce type peut, par exemple, consister en un conservateur de type « Cary-Blair », « Cary-Blair modifié », tel que décrit dans la publication Srijan et al. **[1]**, ou encore un bouillon trypticase soja éventuellement supplémenté avec du glycérol et/ou 5% de sang de mouton (conformément à l'enseignement de la publication de Wasfy et al. **[2]**).

Evidemment, l'homme du métier saura adapter tant la nature que le volume de la solution de mise en suspension en fonction de la nature et du volume d'échantillon prélevé.

Avantageusement, ladite solution de mise en suspension comprend un calibrant du type ADN de plante, bactérie issue de plantes ou peptides lourds, etc..., permettant de suivre et de contrôler l'efficacité des étapes de mise en suspension, de filtration et de lyse (hors du dispositif). En outre, l'utilisation dudit calibrant est particulièrement intéressante pour valider et interpréter les résultats. De surcroît, l'utilisation dudit calibrant directement avec la solution de mise en suspension permet de s'affranchir d'une étape de pipetage supplémentaire dans le protocole.

La solution de mise en suspension peut également comprendre un moyen de capture d'éléments non ciblés (tels que des inhibiteurs, susceptibles d'interférer avec les étapes d'analyses ultérieures). A titre d'exemple, le charbon actif ou le polyvinylpolypyrrolidone (PVPP) peuvent être utilisés en tant que moyen de capture d'éléments non ciblés pour améliorer la sensibilité de détection, en retenant des éléments susceptibles de perturber/d'inhiber les étapes d'analyses ultérieures

Par ailleurs, lorsque l'on souhaite analyser de l'information biologique contenue dans de la matière biologique, de préférence dans de la matière biologique autoreproductible, la solution de mise en suspension telle que définie supra peut, selon un mode de réalisation particulier de l'invention, contenir en outre une solution de lyse, afin d'induire la lyse de ladite matière biologique et la libération de l'information biologique recherchée au sein de ladite solution de mise en suspension d'échantillon.

Selon un mode de réalisation particulièrement préféré de l'invention, ledit moyen de filtration est sélectionné parmi :

- un filtre à gradient, et
- une superposition d'au moins deux filtres, de préférence de deux ou trois filtres, dont la taille des pores décroît de l'intérieur vers l'extérieur du dispositif.

Selon un mode de réalisation préféré, le dispositif est adapté pour contenir un ou plusieurs filtres ayant chacun une surface supérieure à 50 mm², préférentiellement comprise entre 100 et 350 mm², avantageusement comprise entre 290 et 330 mm², afin de limiter le colmatage des filtres tout en assurant un volume filtré maximal.

Par « filtre à gradient » (également dénommé « filtre complexe »), l'on entend un filtre présentant une pluralité de tailles de pores différentes. Plus précisément, lorsqu'un filtre à gradient est employé aux fins de la présente invention, la taille des pores de ce filtre décroît de l'intérieur vers l'extérieur du dispositif afin de permettre une filtration progressive et différentielle de l'échantillon à matrice hétérogène d'intérêt.

En tout état de cause, que l'on utilise un filtre à gradient ou une superposition d'au moins deux filtres, tels que décrits précédemment, la taille des pores décroît de l'intérieur vers l'extérieur du dispositif, de préférence de 500 μm à 0,1 μm, avantageusement de 200 μm à 10 μm, de manière particulièrement préférée de 200 μm à 20 μm. Bien évidemment, la taille des pores du/des filtres utilisé(s) est adaptée en fonction de la taille de la matière biologique et/ou de l'information biologique que l'on souhaite récupérer dans le filtrat.

Ainsi, lorsque l'on souhaite isoler, à partir de l'échantillon prélevé, des virus, toxines, métabolites ou autres informations biologiques présentes au sein dudit échantillon, une taille de pore comprise entre 0,1 μm et 20 μm, préférentiellement 0,1 μm et 5 μm, peut être utilisée.

Dans le cas d'une superposition d'au moins deux filtres, l'on utilise, de préférence, une superposition de deux à quatre filtres. Avantageusement, l'on utilise une superposition de deux ou trois filtres.

Dans un mode de réalisation particulier, ledit moyen de filtration comprend au moins un moyen de capture d'éléments non ciblés (tels que des inhibiteurs, susceptibles d'interférer avec les étapes d'analyses ultérieures). A titre illustratif, ledit moyen de capture d'éléments non ciblés peut consister en du charbon actif, ou en un matériau ayant une affinité particulière pour les inhibiteurs pouvant être contenue dans de la matière biologique (protéines, polysaccharides, etc...), par exemple le PVPP.

Selon un mode de réalisation préféré, le contenant comprend au moins une paroi comportant au moins une zone en matière souple, adaptée pour subir une compression et générer en réponse une surpression à l'intérieur dudit contenant afin de permettre ou faciliter la filtration du contenu au travers dudit moyen de filtration.

Ladite au moins une zone en matière souple s'avère particulièrement avantageuse, dans la mesure où l'opérateur peut générer sur ladite au moins une zone une pression manuelle sans force excessive, afin de permettre/faciliter la filtration du contenu au travers dudit moyen de filtration, tel qu'indiqué précédemment. Le dispositif selon l'invention permet donc à tout(e) technicien(ne) de laboratoire, même les moins expérimenté(e)s, d'obtenir, en mettant en œuvre le dispositif selon l'invention, un filtrat contenant la matière biologique et/ou l'information biologique à analyser. De manière générale, le dispositif selon l'invention peut s'adapter à tout système/dispositif permettant de faciliter l'opération de filtration. Par exemple, ledit dispositif peut être adapté à un système de filtration sous-vide ou à un système de filtration par centrifugation. Il résulte de ce qui précède que le dispositif selon l'invention peut, contrairement au protocole d'obtention de matière biologique et/ou d'information biologique de l'art antérieur, être aisément automatisé, par exemple au moyen d'un automate de filtration (ou dispositif automatique de filtration), également objet de la présente invention. Un tel automate de filtration, adapté pour permettre l'automatisation de l'étape de filtration du contenu (à savoir de la suspension contenant l'échantillon à matrice hétérogène d'intérêt), comprend :

- au moins un emplacement (et de préférence une pluralité d'emplacements) adapté pour recevoir et maintenir le dispositif d'obtention de matière biologique et/ou d'information biologique selon l'invention, de préférence en position « inversée » ou « retournée » (à savoir dans laquelle la susdite ouverture pointe essentiellement vers le sol),
- un organe de pression (par exemple un pressoir tel qu'un piston), mobile d'une position initiale (« position de repos ») vers une position « de pression », dans laquelle ledit organe de pression est adapté pour exercer une pression contre ladite au moins une zone en matière souple, ce qui a pour effet de générer, en réponse, une surpression à l'intérieur dudit contenant et permet ainsi la filtration du contenu au travers dudit moyen de filtration vers un récipient collecteur (par exemple un tube, tel qu'un tube eppendorf®).

De façon optimale, l'automate de filtration selon l'invention comprend jusqu'à huit voire dix emplacements différents, ce qui permet de filtrer simultanément jusqu'à huit voire dix dispositifs d'obtention de matière biologique et/ou d'information biologique selon l'invention.

De préférence, l'organe de pression est actionné par un moteur sur commande de l'utilisateur ou d'un centre de commande automatisé. Par exemple, l'utilisateur met en route le moteur en activant un interrupteur (par exemple en appuyant sur un bouton poussoir), ce qui a pour effet d'induire le passage de l'organe de pression d'une position initiale (« position de repos ») vers une position « de pression », dans laquelle ledit organe de pression exerce une pression contre ladite au moins une zone en matière souple.

Selon un mode de réalisation particulièrement avantageux de l'invention, l'automate de filtration susvisé est pourvu d'un détecteur optique de niveau (également dénommé « barrière optique ») stoppant l'étape de filtration lorsque le niveau souhaité de filtrat est atteint au sein du récipient collecteur. Plus précisément, ce détecteur optique fonctionne en induisant le passage de l'organe de pression de la position de pression vers la position de repos (sous l'action du moteur ou, plus simplement, par arrêt de celui-ci), à savoir stoppe la pression exercée par l'organe de pression sur la zone en matière souple lorsque le détecteur optique de niveau détecte, via un capteur optique, que le niveau souhaité de filtrat est atteint au sein du récipient collecteur, mettant ainsi fin à l'étape de filtration. Sans être lié par la théorie, le fait que l'organe de pression cesse d'exercer une pression contre ladite au moins une zone en matière souple a pour effet de mettre fin à la surpression précédemment générée à l'intérieur du contenant, ce qui conduit l'arrêt de l'étape de filtration lorsque le niveau souhaité de filtrat est atteint au sein du récipient collecteur. Un automate de filtration selon l'invention, pourvu d'un détecteur optique de niveau, s'avère particulièrement avantageux, dans la mesure où il permet de collecter le même volume de filtrat, et ce quel que soit le type de selles, assurant répétabilité et robustesse au procédé d'obtention de matière biologique et/ou d'information biologique mettant en œuvre le dispositif d'obtention de matière biologique et/ou d'information biologique selon l'invention. Ceci est d'autant plus vrai que, comme expliqué précédemment, la partie creuse calibrée du dispositif d'obtention de matière biologique et/ou d'information biologique selon l'invention permet de prélever une masse donnée/prédéfinie d'échantillon à matrice hétérogène (sans avoir à effectuer des opérations de pesée), ladite masse étant constante ou quasi-constante à chaque opération de prélèvement d'échantillon à matrice hétérogène. En d'autres termes, ladite partie creuse calibrée et l'automate de filtration selon l'invention, pourvu d'un détecteur optique de niveau, agissent en synergie afin de garantir une répétabilité et une robustesse optimales au procédé d'obtention de matière biologique et/ou d'information biologique mettant en œuvre le dispositif d'obtention de matière biologique et/ou d'information biologique selon l'invention.

Dans un mode de réalisation préféré, ledit bouchon comprend au moins une zone rigide, ladite zone rigide ayant une forme adaptée pour coopérer avec au moins un organe de maintien, tel qu'une pince, connecté à un appareil de malaxage, tel qu'un vortex, de façon à permettre le maintien dudit dispositif sur l'appareil de malaxage lors du malaxage dudit dispositif afin de faciliter la mise en suspension dudit échantillon dans ladite solution de mise en suspension.

Selon un mode de réalisation préféré, ladite zone rigide ayant une forme adaptée pour coopérer avec une pince connectée audit appareil de malaxage, ladite zone rigide comprenant :

- au moins un moyen anti-rotation, comprenant deux surfaces d'appui distinctes, par exemple constituées par deux épaulements ou par deux languettes, chacune des deux surfaces d'appui distinctes étant adaptée pour être ou venir en butée contre une des deux extrémités de la pince lorsque le bouchon subit un mouvement de rotation, afin d'empêcher ou stopper ladite rotation, et/ou
- au moins un moyen anti-translation, comprenant au moins une surface d'appui, telle qu'un rebord, une collerette ou un épaulement, ladite au moins une surface d'appui étant adaptée pour être ou venir en butée contre ladite pince lorsque le bouchon subit un mouvement de translation, afin d'empêcher ou stopper ledit mouvement de translation.

Ladite au moins une zone rigide permet au dispositif de l'invention de s'adapter aisément aux appareils de malaxage/d'homogénéisation disponibles dans le commerce, tels que des appareils de type vortex, et d'obtenir des résultats à tout le moins équivalents à ceux observés avec des dispositifs spécifiquement conçus pour cet usage, du type broyeur à billes (« bead beater » en langue anglaise).

Le moyen anti-rotation et/ou le moyen anti-translation susvisé(s) permet/permettent d'améliorer le maintien du dispositif selon l'invention par l'organe de maintien (par exemple une pince de l'appareil de malaxage de type vortex).

Le moyen anti-rotation permet de limiter, et de préférence de supprimer, toute rotation dudit bouchon (et donc, par extension, du dispositif selon l'invention) par rapport à l'organe de maintien susvisé. Outre le fait que le moyen anti-rotation permette de s'assurer d'un maintien tout à fait satisfaisant entre le bouchon - et donc le dispositif selon l'invention - et l'organe de maintien de l'appareil de malaxage, ce moyen anti-rotation a également pour fonction d'orienter le bouchon, et par conséquent le moyen de prélèvement calibré, solidaire dudit bouchon, de sorte que la partie creuse calibrée dudit moyen de prélèvement soit orientée vers le bas ou vers le haut, de préférence vers le bas, lorsque le dispositif selon l'invention est positionné sur l'organe de maintien de l'appareil de malaxage.

Concernant ledit moyen anti-translation, celui-ci permet de limiter, et de préférence de supprimer, tout mouvement de translation du bouchon (et donc, par extension, du dispositif selon l'invention) suivant un vecteur orienté dans le sens bouchon-contenant lors de l'opération de malaxage. Ce moyen anti-translation permet donc d'assurer un malaxage efficace et d'éviter que le dispositif selon l'invention ne s'échappe de l'organe de maintien lors de ladite opération de malaxage. Avantageusement, ce moyen anti-translation comprend au moins un rebord (partie en saillie) positionné sur tout ou partie du pourtour de la partie supérieure du bouchon.

Selon un mode de réalisation particulier, ce moyen anti-translation consiste en une collerette (partie circulaire) positionnée sur la partie supérieure du bouchon et connectée à cette dernière, de diamètre supérieur au diamètre du corps du bouchon.

Le rebord formé sur tout ou partie du pourtour de la partie supérieure du bouchon constitue une face d'appui, laquelle va coopérer avec l'organe de maintien susvisé (tel qu'une pince) afin de garantir le maintien du bouchon - et donc du dispositif selon l'invention - lors de l'opération de malaxage. Selon un mode de réalisation préféré, lorsque l'organe de maintien est une pince, la face d'appui constitué par le rebord ou la collerette, tel que mentionné supra, va venir en butée contre ladite pince afin d'empêcher ou stopper tout mouvement de translation, comme expliqué ci-après, sous l'intitulé « description détaillée de l'invention ».

Selon un mode de réalisation préféré de l'invention, la partie supérieure du contenant - avantageusement l'épaulement du flacon dans l'éventualité où ledit contenant est un flacon - remplit la fonction de moyen anti-translation en permettant de limiter, voire supprimer, tout mouvement de translation du bouchon (et donc, par extension, du dispositif selon l'invention) suivant un vecteur orienté dans le sens contenant-bouchon lors de l'opération de malaxage. En effet, la face d'appui constitué par la partie supérieure dudit contenant (par exemple l'épaulement du flacon) est/vient en butée contre ledit organe de maintien afin d'empêcher ou stopper tout mouvement de translation du dispositif selon l'invention suivant un vecteur orienté dans le sens contenant-bouchon.

Selon une variante de l'invention, un deuxième moyen anti-translation peut être positionné sur tout ou partie du pourtour de la partie inférieure du bouchon, afin de limiter, voire supprimer, tout mouvement de translation du bouchon (et donc, par extension, du dispositif selon l'invention) suivant un vecteur orienté dans le sens contenant-bouchon lors de l'opération de malaxage.

D'une manière générale, ledit dispositif est un dispositif d'obtention de matière biologique, de préférence de matière microbiologique, et ledit moyen de filtration est adapté pour permettre le passage sélectif de ladite matière biologique, de préférence de ladite matière microbiologique, vers l'extérieur dudit dispositif.

L'invention a également pour objet le bouchon *perse* tel que défini précédemment.

En outre, l'invention concerne également l'utilisation du dispositif susvisé pour l'obtention de matière biologique et/ou d'information biologique à partir d'un échantillon à matrice hétérogène, de préférence pour l'obtention de matière biologique, préférablement pour l'obtention de matière biologique microscopique, avantageusement pour l'obtention de matière microbiologique. Ledit moyen de filtration est adapté en conséquence, afin de permettre le passage sélectif de ladite matière biologique, de préférence de ladite matière biologique microscopique, avantageusement de ladite matière microbiologique, vers l'extérieur dudit dispositif.

Selon un mode de réalisation préféré, et tel qu'indiqué précédemment, l'échantillon à matrice hétérogène est sélectionné parmi un échantillon de sol, un échantillon de selles, un échantillon de tissu nécrosé, un échantillon alimentaire, un échantillon industriel, de préférence ledit échantillon étant un échantillon de selles humaines ou animales.

Avantageusement, ledit échantillon à matrice hétérogène est un échantillon de selles.

Un autre objet de l'invention concerne un procédé d'obtention de matière biologique et/ou d'information biologique à partir d'un échantillon à matrice hétérogène, ledit procédé mettant en œuvre le dispositif selon ledit procédé comprenant les étapes suivantes :

a) prélever un volume d'échantillon prédéterminé correspondant à une masse donnée à l'aide dudit moyen de prélèvement calibré,

b) mettre en suspension ledit échantillon dans ladite solution de mise en suspension, éventuellement par mise en suspension mécanique,

c) filtrer la suspension obtenue à l'étape b) à travers ledit moyen de filtration afin d'obtenir un filtrat contenant ladite matière biologique et/ou ladite information biologique.

Selon un mode de réalisation particulier, l'opérateur peut stocker la suspension obtenue à l'issue de l'étape b) susvisée et, le cas échéant, différer dans le temps l'étape de filtration c). Ceci permet notamment de conserver la matière biologique et/ou l'information biologique d'intérêt aux fins d'analyse(s) ultérieure(s) ou, dans le cas de prélèvements effectués « sur le terrain » (à savoir hors du laboratoire), d'adresser à un laboratoire le dispositif selon l'invention comprenant la suspension obtenue à l'étape b), à savoir comportant la matière biologique et/ou l'information biologique d'intérêt, obtenue(s) à partir du prélèvement d'échantillon à matrice hétérogène. Ceci s'avère, en effet, particulièrement avantageux lorsque les prélèvements d'échantillon à matrice hétérogène sont effectués dans des zones reculées (par exemple dans des zones de brousse), distantes de tout laboratoire d'analyse. Dans ce mode de réalisation, l'on utilise, de préférence, au moins un moyen de conservation adapté à la nature de la matière biologique et/ou de l'information biologique à analyser. Ainsi, ladite solution de mise en suspension peut, par exemple, contenir un ou plusieurs conservateur(s) chimique(s) (par exemple du type Cary-Blair) et/ou un ou plusieurs moyen(s) de congélation (permettant, à titre illustratif, la congélation à une température de l'ordre de -80°C). Bien évidemment, la condition sine qua none est que le ou les moyen(s) de conservation utilisé(s), n'altère(nt) pas (ou, le cas échéant, de manière peu significative) la matière biologique et/ou l'information biologique à analyser.

Selon un mode de réalisation particulier, ladite solution de mise en suspension comprend au moins un moyen de culture. Ce moyen de culture peut être au moins un élément nutritif ou une pluralité d'éléments nutritifs (par exemple un milieu de culture) permettant de favoriser la croissance de l'ensemble de micro-organismes présents dans l'échantillon prélevé ou d'orienter, de manière plus ou moins sélective, la croissance d'un ou plusieurs type(s) de micro-organismes.

Ledit au moins un moyen de culture peut être également au moins un agent sélectif possédant des propriétés inhibitrices quant à la croissance d'au moins un type de micro-organismes, tel qu'un antibiotique (bactéricide ou bactériostatique, de préférence bactéricide), un antifongique. L'utilisation d'au moins un agent sélectif peut permettre d'orienter la croissance d'au moins un type de micro-organismes.

Bien évidemment, l'homme du métier adaptera le ou les moyen(s) de culture présent(s) dans la solution de mise en suspension en fonction du/des type(s) de micro-organismes d'intérêt.

Un autre objet de l'invention concerne un procédé d'extraction d'information biologique (par exemple d'acides nucléiques, tels que de l'ADN et/ou de l'ARN) à partir d'un échantillon à matrice hétérogène, ledit procédé comprenant les étapes suivantes :

a) mettre en œuvre le procédé d'obtention de matière et/ou d'information biologique susvisé afin d'obtenir un filtrat contenant ladite matière biologique et/ou ladite information biologique,

b) lorsque l'information biologique devant être extraite est contenue au sein de ladite matière biologique, telle qu'une cellule, une bactérie, un champignon ou une levure, réaliser une étape de lyse de ladite matière biologique, de préférence une étape de lyse mécanique, afin d'obtenir un lysat comprenant l'information biologique devant être extraite,

c) extraire l'information biologique à partir du filtrat obtenu à l'étape a) ou du lysat obtenu à l'étape b) en mettant en œuvre un procédé d'extraction d'information biologique adapté.

Selon un mode de réalisation préféré, ce procédé d'extraction est un procédé d'extraction d'acide(s) nucléique(s) (information génétique). Dans ce mode de réalisation, l'étape de lyse b) du procédé d'extraction susvisée peut être intégrée à l'étape d'extraction c). Par exemple, certains systèmes automatisés d'extraction d'information génétique de type easyMAG® (bioMérieux) intègrent une étape de lyse au protocole d'extraction. Ladite lyse peut notamment être de type chimique, mécanique ou thermique, en fonction de la nature d'échantillon à matrice hétérogène, de la matière biologique et/ou information biologique recherchée(s) ou encore des techniques d'analyses qui seront employées ultérieurement.

Selon un mode de réalisation préféré, ledit procédé d'extraction d'information biologique comprend avant l'étape c), et avant l'étape b) lorsque celle-ci doit être effectuée, une étape de concentration de ladite matière biologique et/ou de ladite information biologique, de préférence par centrifugation (avec ou sans coussin de densité type chlorure de Césium par exemple) ou par floculation. Lorsque ladite étape de concentration est effectuée par centrifugation, celle-ci est réalisée à une vitesse comprise entre 6000g et 12000g, avantageusement d'environ 12000g.

Un autre objet de l'invention concerne un procédé d'analyse d'information biologique, ledit procédé comprenant les étapes suivantes :

a) extraire ladite information biologique à analyser en utilisant le susdit procédé d'extraction d'information biologique,
b) identifier et/ou quantifier ladite information biologique par toute méthode d'analyse appropriée, par exemple une méthode d'analyse génétique telle qu'une PCR, une méthode de type immunodosage ou essai enzymatique.

Selon un mode de réalisation préféré, ce procédé d'analyse est un procédé d'analyse génétique (analyse d'acide(s) nucléique(s)). Dans ce cas de figure, ladite information biologique est une information génétique et l'identification et/ou la quantification de cette information génétique est/sont opérée(s) par toute méthode d'analyse génétique appropriée, par exemple par PCR.

En outre, une étape de purification peut, au besoin, être réalisée avant l'étape d), afin d'éliminer tout élément non ciblé (tel qu'un ou plusieurs inhibiteur(s) susceptible(s) d'interférer avec la ou les méthode(s) d'analyse utilisée(s) à l'étape d)).

Un autre objet de l'invention concerne un procédé d'analyse de matière biologique à partir d'un échantillon à matrice hétérogène, ledit procédé comprenant les étapes suivantes :

a) obtenir un filtrat contenant la matière biologique à analyser en utilisant le susdit procédé d'obtention de matière biologique,
b) le cas échéant, inoculer un milieu réactionnel avec le filtrat obtenu à l'étape a), ledit milieu réactionnel étant adapté pour permettre la croissance et/ou l'expression d'au moins un métabolisme de ladite matière biologique,
c) le cas échant, incuber le filtrat obtenu à l'étape a), ou le milieu réactionnel inoculé obtenu à l'étape b), durant un laps de temps et à une température appropriés,
d) analyser la matière biologique à l'issue de l'étape a), b) ou c) par tout moyen d'analyse biologique approprié.

Selon un mode de réalisation préféré, ladite matière biologique est une matière microbiologique.

Dans un mode de réalisation particulier, l'étape a) du procédé d'analyse de matière biologique peut être adaptée pour l'analyse de matières biologiques dites fragiles, telles que des bactéries à Gram-, notamment en utilisant des moyens de mise en suspension entrainant moins de contraintes mécaniques sur les parois bactériennes. Afin de permettre une mise en suspension moins dégradante pour ces bactéries, l'opérateur peut réduire la fréquence de malaxage, utiliser des moyens de suspension mécanique ayant une taille plus importante, ou encore supprimer l'utilisation de moyen de suspension mécanique. Dans le cas où un moyen de suspension mécanique ne serait pas utilisé, et où l'échantillon serait difficile à mettre en suspension, une partie creuse calibrée présentant au moins une ouverture - de préférence une pluralité d'ouvertures - peut être utilisée afin de faciliter la mise en suspension. De manière évidente l'homme du métier saura adapter ces paramètres en fonction de la matière biologique d'intérêt dite « sensible » et de la nature de l'échantillon.

Préférentiellement, le procédé d'analyse de matière microbiologique susvisé comprend l'étape b) et éventuellement l'étape c), avantageusement les étapes b) et c), dans lequel :

- l'étape c), lorsqu'elle est présente, consiste à incuber le milieu réactionnel inoculé obtenu à l'étape b) durant un laps de temps et à une température appropriés, et
- l'étape d'analyse de matière biologique d) consiste en la détection et/ou l'identification et/ou le dénombrement de ladite matière biologique sur/dans ledit milieu réactionnel, de préférence par lecture visuelle ou optique.

Un autre objet de l'invention concerne un kit d'obtention de matière biologique et/ou d'information biologique à partir d'un échantillon à matrice hétérogène, ledit kit comprenant :

- ledit contenant et ledit bouchon, sous forme assemblée ou désassemblée,
- au moins une solution de mise en suspension, adaptée pour permettre la mise en suspension dudit échantillon,
- au moins un moyen de mise en suspension mécanique, tel qu'au moins une bille.

Avantageusement, ledit contenant et ledit bouchon se présentent sous forme désassemblée et sont conditionnés, de préférence, de manière stérile.

Un autre objet de l'invention concerne l'utilisation du kit susvisé pour la mise en œuvre dudit procédé d'obtention de matière biologique et/ou d'information biologique.

Selon un mode préféré de la présente invention, le dispositif selon l'invention est adapté pour la prise d'un échantillon de matière fécale en vue de son analyse microbiologique.

La matière biologique et/ou information biologique présente(s) dans le filtrat obtenu en mettant en œuvre le dispositif selon l'invention peut/peuvent être analysée(s) par diverses méthodes d'analyse biologique, telles que des essais enzymatiques ou immunologiques, des amplifications de séquences nucléiques ou des tests sur boîte de Pétri ou encore par séquençage. En outre, la matière biologique et/ou information biologique présente(s) dans le susdit filtrat peut/peuvent, le cas échéant, être concentrée(s), purifiée(s), lysée(s) ou cultivée(s), enrichie(s) ou être directement analysée(s).

A titre d'exemple, la matière biologique et/ou information biologique présente(s) dans le susdit filtrat peut(vent) être directement analysée(s) par spectrométrie de masse de type MALDI-TOF (dépôt d'au moins une goutte de filtrat sur plaque MALDI-TOF), par spectroscopie Raman, par immuno-chromatographie sur membrane ou bandelette (test par flux latéral ou « latéral flow test » en langue anglaise), ou par des systèmes d'identification bactérienne du type galeries API.

**Brève description des dessins**

L'invention, sa fonctionnalité, ses applications ainsi que ses avantages seront mieux appréhendés à la lecture de la présente description, faite en référence aux figures, dans lesquelles :

- les figures 1A et 1B montrent respectivement une vue de face et une vue en coupe d'un premier mode de réalisation du dispositif selon la présente invention,
- la figure 2 représente une vue de face du bouchon, désolidarisé du contenant tel que représenté sur les figure 1A et 1B,
- la figure 3 est une vue de dessous du bouchon de la figure 2,
- la figure 4 est une vue de face d'un bouchon selon un deuxième mode de réalisation, désolidarisé du contenant selon l'invention,
- la figure 5 représente une vue de dessous du bouchon représenté sur la figure 4,
- la figure 6 représente une vue de côté d'un dispositif selon l'invention connecté à un appareil de malaxage par l'intermédiaire d'une pince,
- la figure 7 est une vue de face de l'ensemble dispositif selon l'invention-pince-appareil de malaxage, tel que représenté en figure 6,
- les figures 8A et 8B sont des vues en perspective d'un bouchon selon un troisième mode de réalisation,
- la figure 9 montre une vue de face du bec verseur du bouchon représenté sur les figures 8A et 8B,
- la figure 10 est une vue en perspective du plan supérieur de la partie centrale du bouchon représenté sur les figures 8A et 8B,
- la figure 11 représente une vue en perspective du plan inférieur de la partie centrale du bouchon représenté sur les figures 8A et 8B,
- la figure 12 représente une vue de face d'un deuxième mode de réalisation du bec verseur du bouchon représenté sur les figures 8A et 8B,
- les figures 13A et 13B représentent deux vues en perspective du moyen de prélèvement adapté pour être connecté au bouchon représenté sur les figures 8A et 8B,
- la figure 14 représente une vue en perspective d'un deuxième mode de réalisation du moyen de prélèvement calibré selon l'invention,
- la figure 15 montre une vue en perspective d'un troisième mode de réalisation du moyen de prélèvement calibré selon l'invention,
- la figure 16 est une vue en perspective d'un bouchon comprenant un quatrième mode de réalisation du moyen de prélèvement calibré selon l'invention,
- les figures 17 et 18 représentent respectivement une vue de face et une vue de côté d'un cinquième mode de réalisation du moyen de prélèvement calibré selon l'invention, comprenant une extension coulissante en position « sortie », à savoir conférant à la tige une première longueur de tige, tel que décrit précédemment,
- les figures 19 et 20 représentent respectivement une vue de face et une vue de côté du susdit cinquième mode de réalisation du moyen de prélèvement calibré selon l'invention mais dans lequel l'extension coulissante en position « rentrée », à savoir conférant à la tige une deuxième longueur de tige, tel que décrit précédemment, inférieure à la susdite première longueur de tige,
- la figure 21 est une vue en coupe d'un automate de filtration selon l'invention, adapté pour permettre l'automatisation de l'étape de filtration du contenu (à savoir de la suspension contenant l'échantillon à matrice hétérogène d'intérêt) à partir du dispositif d'obtention de matière biologique et/ou d'information biologique selon l'invention,
- la figure 22 est un diagramme schématisant les différentes étapes des procédés d'analyse d'information biologique et de matière biologique selon la présente invention,
- la figure 23 est une représentation graphique des résultats obtenus pour un test de linéarité, quantifiant l'ADN (par PCR, Nombre de Cq) des bactéries à Gram négatif (KPC) par le procédé d'analyse d'information biologique selon l'invention, en fonction du log de la concentration en KPC inoculée. Une régression linéaire ($R^2$) est représentée et

calculée (Linéaire).

- la figure 24 est une représentation graphique des résultats obtenus pour un test de linéarité, quantifiant l'ADN (par PCR, Nombre de Cq) des bactéries à Gram positif (MRSA) par le procédé d'analyse d'information biologique selon l'invention, en fonction du log de la concentration en MRSA inoculée. Une régression linéaire ($R^2$) est représentée et calculée (Linéaire).
- la figure 25 est une représentation graphique des résultats obtenus pour un test de linéarité, quantifiant l'ADN (par PCR, Nombre de Cq) de levures S. *pombe* par le procédé d'analyse d'information biologique selon l'invention, en fonction du log de la concentration en S. *pombe* inoculée. Une régression linéaire ($R^2$) est représentée et calculée (Linéaire).
- la figure 26 compare la quantification par PCR d'ADN d'adénovirus en utilisant le kit Adenovirus R-gene® (Référence : 69-010B) avec le kit d'extraction QIAamp® DNA stool (QiaGen) et le procédé d'extraction d'information biologique selon l'invention.
- la figure 27 (à gauche échelle en ng/ μL, à droite en ratio) présente l'impact du type Bristol des selles sur la quantité et la pureté de l'ADN de micro-organismes extrait.

## Description détaillée de l'invention

La description détaillée ci-après a pour but d'exposer l'invention de manière suffisamment claire et complète, notamment en référence aux figures susvisées, mais ne doit en aucun cas être regardée comme limitant l'étendue de la protection aux modes de réalisation particuliers objet desdites figures.

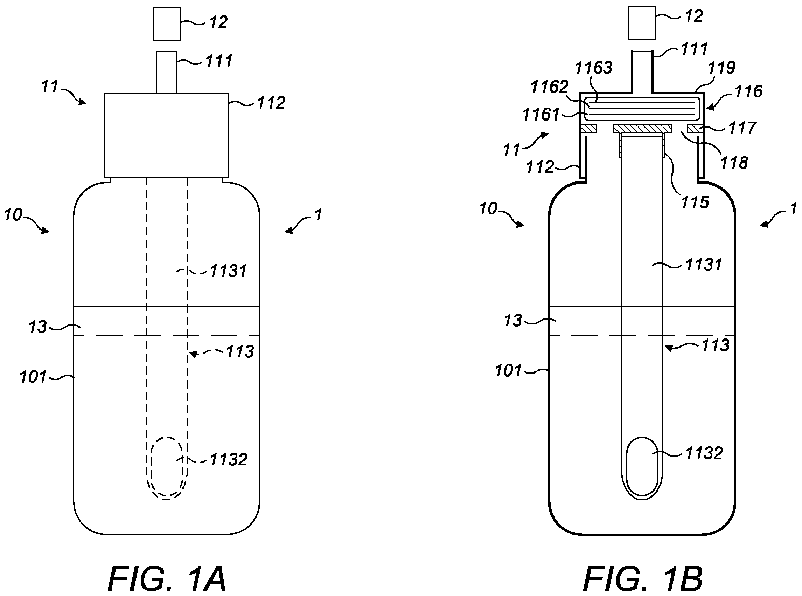

Comme représenté sur la figure 1A, le dispositif selon l'invention comprend un contenant 10, se présentant sous la forme d'un flacon en plastique souple, pouvant être déformé notamment via une pression exercée sur ledit contenant par les doigts de l'opérateur.

La paroi souple 101 du susdit contenant 10 est en matériau souple. Cette paroi souple 101 contient, par exemple, du papier rigide, du carton, du polyéthylène, du polychlorure de vinyle, du polypropylène, du polyéthylène basse densité, du polyéthylène téréphtalate, ainsi que toute combinaison adaptée de ces matériaux et/ou de tout autre matériau biosourcé afin que la paroi souple 101 du contenant 10 possède des propriétés satisfaisantes notamment en matière de rigidité (afin de pouvoir poser le contenant sur une surface plane), d'étanchéité et de déformation par compression lorsqu'une pression est exercée (par exemple par les doigts de l'opérateur ou par l'organe de pression d'un automate de filtration, tel que décrit ci-dessus et ci-après) sur la paroi souple 101 du contenant 10, lors de l'étape de filtration.

Comme représenté sur cette figure 1A, le contenant 10 est fermé par l'intermédiaire d'un bouchon 11 comprenant, de sa partie supérieure vers sa partie inférieure :

- un orifice de forme tubulaire 111, permettant *in fine* de récolter le filtrat (obturable par un capuchon 12),
- un corps de bouchon 112 (partie centrale),
- un moyen de prélèvement calibré 113 connecté à la partie inférieure du bouchon 11 (non numérotée sur la figure 1A).

Plus précisément, ce moyen de prélèvement calibrée 113 comprend une partie creuse calibrée 1132 reliée à la partie inférieure du bouchon (non numérotée sur la figure 1A) par l'intermédiaire d'une tige 1131. Le volume de la partie creuse calibrée 1132 a été calculé au préalable pour correspondre à une masse prédéterminée d'échantillon à matrice hétérogène, susceptible de contenir la matière biologique et/ou l'information biologique d'intérêt.

Le dispositif 1 est constitué par assemblage du bouchon 11 et du contenant 10.

Le contenant 10 est rempli avec un volume suffisant de solution de mise en suspension 13, telle que définie précédemment. Le niveau de solution de mise en suspension 13 est représenté sur les figures 1A et 1B à titre purement indicatif.

La structure interne du bouchon 11 - et en particulier du corps de bouchon 112 - est bien visible sur la figure 1B, laquelle représente une vue en coupe du dispositif 1. Le corps du bouchon 112 comprend une partie interne 117 et une partie externe 119, ces deux parties 117 et 119 délimitant une cavité au sein de laquelle est disposé un moyen de filtration 116. Ce moyen de filtration 116 est constitué d'une superposition de trois filtres 1161, 1162 et 1163, ayant chacun une surface de 314 mm²; chacun des trois filtres 1161, 1162 et 1163 possédant en outre des pores de tailles dégressives de la partie interne 117 vers la partie externe 119 du corps de bouchon 112. Plus précisément, le premier filtre 1161 possède des pores dont la taille est comprise entre 100 μm et 200 μm alors que les deux filtres « supérieurs » 1162 et 1163 ont chacun des pores de taille inférieure à 50 μm. Le premier filtre 1161 permet d'éliminer les plus grosses entités (par exemple débris cellulaires, etc.) alors que les deux filtres «supérieurs» 1162 et 1163 permettent d'assurer la sélectivité de l'opération de filtration. Tel qu'illustré en figure 1B, le filtre 1163 possédant les tailles de pores les plus faibles (à égalité avec celles du filtre 1162) vient en contact avec la partie externe 119 du corps du bouchon 112.

La tige 1131 du moyen de prélèvement calibré 113 est connectée à la partie interne 117 du bouchon 11 via un moyen de connexion (ou de fixation) 115, positionné sur ladite partie interne 117.

Des ouvertures 118 sont pratiquées au sein de la partie 117 du bouchon 11 afin de permettre le passage de la solution de mise en suspension 13 comprenant l'échantillon à matrice hétérogène (non représenté) vers l'orifice de forme tubulaire 111 via le moyen de filtration 116, comprenant la superposition des trois filtres 1161, 1162 et 1163.

A des fins de clarté, l'utilisation du dispositif 1 selon l'invention est brièvement décrite ci-après.

L'opérateur prélève, à l'aide de la partie creuse 1132 du moyen de prélèvement calibré 113, un volume correspondant au volume défini par la partie creuse calibrée 1132 et correspondant à une masse d'échantillon à matrice hétérogène souhaitée. Le contenant 10 à paroi souple 101 est rempli avec un volume suffisant d'une solution de mise en suspension 13. Comme illustré sur les figures 1A et 1B, le contenant 10 est fermé/obturé par le bouchon 11 (par exemple le bouchon 11 est vissé sur un pas de vis positionné sur le col dudit contenant) et, de manière optimale, l'orifice de forme tubulaire 111 est obturé par le capuchon 12.

Le dispositif 1 est ensuite agité afin de permettre la mise en suspension de l'échantillon à matrice hétérogène contenu dans la partie creuse calibrée 1132 du moyen de prélèvement 113. Une fois la mise en suspension réalisée, l'opérateur enlève, le cas échéant, le capuchon 12 et « retourne » le dispositif 1 (ce dernier étant donc dans une position dite « inversée » ou « retournée », à savoir dans laquelle l'orifice de forme tubulaire 111 pointe essentiellement vers le sol). Du fait de cette opération de retournement, la solution de mise en suspension 13, chargée en échantillon à matrice hétérogène, passe au travers des ouvertures 118 pratiquées au sein de la partie interne 117 du bouchon 11, traverse successivement les trois filtres 1161, 1162 et 1163 du moyen de filtration et s'écoule vers l'extérieur, sous forme de filtrat, via l'orifice de forme tubulaire 111. Le filtrat est ensuite collecté dans tout type de récipient approprié, par exemple dans un tube Eppendorf.

De manière avantageuse, et tel qu'indiqué précédemment, le passage de la solution de mise en suspension 13, chargée en échantillon à matrice hétérogène, à travers le moyen de filtration 116 est facilité/accéléré lorsque l'opérateur exerce une pression sur la paroi souple 101 du contenant 10, résultant en une surpression au sein dudit contenant 10 et notamment au niveau du moyen de filtration 116. Cette opération de compression de la paroi souple 101 par l'opérateur s'avère particulièrement avantageuse pour faciliter la filtration, étant donné la petitesse/l'étroitesse des pores des deux filtres supérieurs 1162 et 1163.

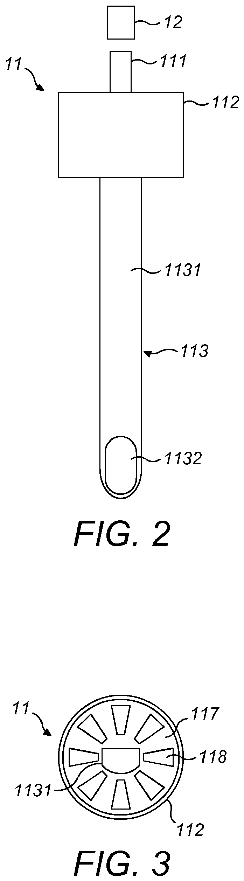

La vue représentée en figure 2 permet de visualiser le bouchon 11, lorsque celui-ci est désassemblé du contenant 10, par exemple par dévissage dudit bouchon 11. Sur cette figure 2, l'on observe distinctement l'orifice de forme tubulaire 111, obturable à l'aide du capuchon 12, le corps de bouchon 112, le moyen de prélèvement calibré 113. Comme représenté sur les figures 1A et 1B, ce dernier comprend une tige 1131 connectée, par l'une de ses extrémités, à la partie interne du corps de bouchon 112 et comprenant, au niveau de son extrémité opposée, une partie creuse calibrée 1132.

Tel qu'indiqué précédemment, la figure 3 est une vue de dessous du bouchon 11. L'on visualise clairement, sur cette figure 3, la disposition radiale des ouvertures 118, pratiquées au sein de la partie interne 117 du bouchon 11, autour de la tige 1131 du moyen de prélèvement 113. Bien que le nombre et la disposition des ouvertures 118 puissent varier en fonction des desiderata de l'homme du métier, la disposition radiale des ouvertures 118 telle que représentée en figure 3 permet une bonne répartition du volume de la solution de mise en suspension 13 chargée en échantillon à matrice hétérogène vers le moyen de filtration (non représenté sur la figure 3) via lesdites ouvertures 118.

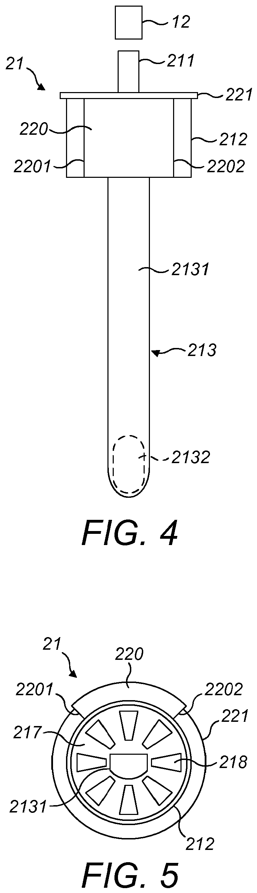

La figure 4 illustre un deuxième mode de réalisation d'un bouchon 21 selon l'invention. Le corps de bouchon 212 est pourvu :

- d'un moyen anti-rotation 220, et
- d'un moyen anti-translation 221, tous deux adaptés pour coopérer avec un organe de maintien tel qu'une pince (non représentée), d'un appareil de malaxage, tel qu'un vortex (également non représenté en figure 4).

Le moyen anti-rotation 220 est positionné sur la surface extérieure du corps de bouchon 21. Tel que représenté sur la figure 4, le moyen anti-rotation 220 est constitué par une surépaisseur à la surface du corps de bouchon 21. Cette surépaisseur définit deux épaulements 2201 et 2202 sur le corps de bouchon 212.

Lorsque l'organe de maintien de l'appareil de malaxage (non représenté sur la figure 4) est une pince, chacune des deux branches de cette pince va venir se positionner de part et d'autre du moyen anti-rotation 220. Lorsqu'un mouvement de rotation est impulsé au bouchon 21 lors de l'étape de malaxage, un des deux épaulements 2201 ou 2202 (en fonction du sens de rotation) va venir buter sur l'extrémité d'une des deux branches de la pince, empêchant ou stoppant de facto la rotation dudit bouchon 21. De préférence, le bouchon est positionné sur la pince de sorte que les épaulements 2201 et 2202 viennent en butée contre les extrémités de chacune des deux branches de la pince.

Ce moyen anti-rotation s'avère particulièrement avantageux, dans la mesure où il permet d'imposer une orientation particulière au moyen de prélèvement calibré 213 connecté à la partie interne (non représentée en figure 4) du bouchon 21. Ainsi, l'élément anti-rotation 220 est positionné sur la surface externe du corps de bouchon 212 de manière à ce que la partie creuse calibrée 2132 du moyen de prélèvement 213 soit orientée vers le bas lorsque le dispositif comprenant le bouchon 21 est disposé sur l'organe de maintien (par exemple une pince) de l'appareil de malaxage de type vortex. Cette orientation de la partie creuse « vers le bas » permet d'améliorer la mise en suspension de l'échantillon à matrice hétérogène qu'elle contient.

Le moyen anti-translation 221, tel que représenté sur la figure 4, est constitué par une collerette de diamètre supérieur à celui du corps de bouchon 212. La fonction de ce moyen anti-translation 221 sera décrite ci-après, en lien avec la figure 5.

L'agencement des moyens anti-rotation 220 et anti-translation 221 par rapport au corps de bouchon 212, et en particulier à la partie interne 217 de ce corps de bouchon 212 est clairement illustré en figure 5, laquelle représente une vue de dessous du bouchon 21 selon le deuxième mode de réalisation de l'invention, ledit bouchon 21 étant désolidarisé du contenant 10. Comme cela est le cas en figure 3, l'on observe, là encore, la disposition radiale des ouvertures 218 autour de la tige 2131 du moyen de prélèvement 213.

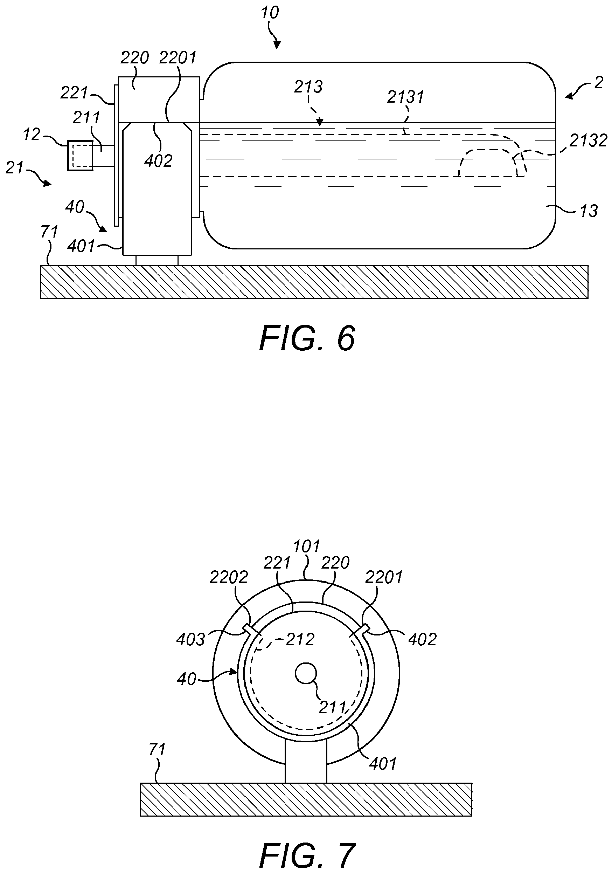

La figure 6 permet de visualiser le maintien du dispositif 2 (comprenant le bouchon 21 selon un deuxième mode de réalisation et le contenant 10) sur un appareil de malaxage 71 via la coopération du corps de bouchon 212 et d'une pince 40, connectée à l'appareil de malaxage 71. A cet égard, il convient de noter que la pince 40 peut faire partie intégrante de l'appareil de malaxage 71 ou peut être connectée sur celui-ci par tout moyen approprié. De manière plus précise, l'on observe sur cette figure 6, que l'épaulement 2201 du moyen anti-rotation 220 vient en butée contre l'extrémité 402 de la branche correspondante de la pince 40. Bien évidemment, l'épaulement 2202 vient également en butée contre l'extrémité 403 de la branche correspondante de la pince 40, même si cela ne peut être observé sur la figure 6. En d'autres termes, le moyen anti-rotation 220 « bute » sur chacune des deux extrémités 402 et 403 de la pince 40, de sorte que le bouchon 21, et par conséquent le dispositif 2, ne peut subir aucun mouvement de rotation, dans un sens ou dans l'autre. De manière tout à fait intéressante, l'on constate, sur cette figure 6, que le moyen anti-rotation 220 a été positionné sur la surface externe du corps de bouchon 212 de manière à orienter la partie creuse calibrée 2132 «vers le bas » lorsque le bouchon 21 est maintenu par la pince 40. Tel qu'indiqué précédemment, l'orientation de la partie creuse calibrée 2132 vers le bas permet d'assurer une mise en suspension optimale de l'échantillon à matrice hétérogène.

En outre, le moyen anti-translation 221 est également bien visible sur la figure 6. Tel qu'indiqué précédemment, il s'agit, dans ce mode de réalisation, d'une collerette de diamètre supérieur à celui du corps de bouchon 212. Lorsqu'un mouvement de translation se produit suivant un vecteur orienté dans le sens bouchon 21-contenant 10, la collerette joue le rôle de face d'appui, laquelle vient en butée contre la partie 401 de la pince 40, empêchant ou stoppant ainsi ce mouvement de translation.

Comme représenté sur la figure 6, le contenant 10 est rempli avec un volume suffisant de solution de mise en suspension 13, telle que définie précédemment. Le niveau de cette solution de mise en suspension 13 est représenté à titre purement indicatif sur la figure 6. L'orifice de forme tubulaire 221 du bouchon 21 est en outre obturé par le capuchon 12, afin d'éviter toute sortie intempestive de la solution de mise en suspension 13 lors de l'étape de malaxage.

La figure 7 représente une vue de face du bouchon 21, maintenu par la pince 40, elle-même connectée à l'appareil de malaxage 71. Sur cette figure 6, l'on distingue nettement le positionnement de la pince 40 de part et d'autre du moyen anti-rotation 220, de sorte que chacun des deux épaulements 2201 et 2202 viennent en butée contre les extrémités correspondantes 402 et 403 de chacune des deux branches de la pince 40.

Cette pince 40 est en outre positionnée sous le moyen anti-translation 221. Dans l'éventualité d'un mouvement de type translation (suivant un vecteur orienté dans le sens bouchon 21-contenant 10), la collerette constituant le moyen anti-translation 221 va venir en butée contre la partie 401 (non représentée sur la figure 7) de la pince 40 pour empêcher ou stopper tout mouvement de translation.

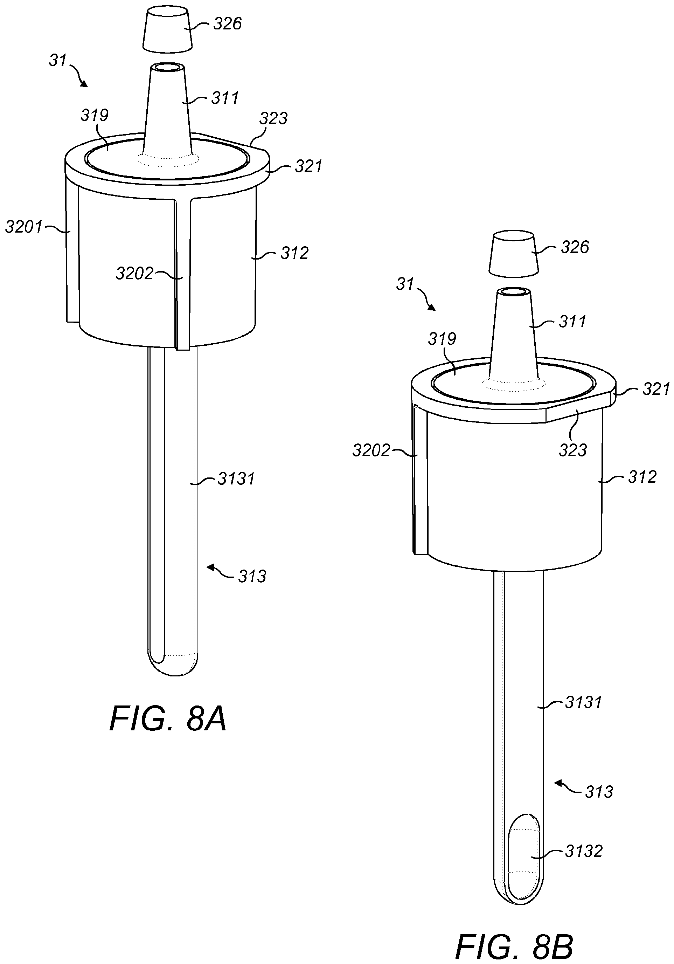

Un troisième mode de réalisation du bouchon 31 est illustré en figures 8A et 8B. Ces dernières représentent clairement ce bouchon 31, lequel comprend, de la partie supérieure vers la partie inférieure :

- un orifice de forme tubulaire 311,
- une partie supérieure 319,
- un moyen anti-translation 321,
- un corps de bouchon 312,
- un moyen anti-rotation 3201, 3202
- un moyen de prélèvement calibré 313 comprenant une tige 3131 et une partie creuse 3132.

En outre, l'orifice de forme tubulaire 311 est obturable au moyen du capuchon 326.

Comme il est possible de le constater à la lumière des figures 8A et 8B, cette partie creuse 3132 est orientée de manière opposée au moyen anti-rotation 3201, 3202. De cette manière, lorsque le dispositif comprenant le bouchon 31 et le contenant 10 est maintenu par une pince connectée à un appareil de malaxage, le moyen anti-rotation 3201, 3202 se trouve orienté vers le haut, comme illustré en figure 6 alors que ladite partie creuse calibrée 3132 est délibérément orientée vers le bas, afin de favoriser la mise en suspension de l'échantillon à matrice hétérogène présent dans la partie creuse calibrée 3132.

En outre, contrairement au bouchon selon un deuxième mode de réalisation de l'invention, représenté aux figures 4-7, dans lequel le moyen anti-rotation 3201, 3202 est constitué par une surépaisseur de matériau, (par exemple une surépaisseur de matériau plastique), positionnée sur la largeur d'une partie du corps du bouchon 212, le moyen anti-translation 3201, 3202 du bouchon 31 comprend, sur toute la longueur du corps du bouchon 312, deux languettes parallèles 3201, 3202, présentes à la surface du corps de bouchon 312.

Comme pour les épaulements 2201 et 2202 représentés en figure 5, lorsqu'un mouvement de rotation est impulsé au bouchon 21 lors de l'étape de malaxage, une des deux languettes 3201, 3202 (en fonction du sens de rotation) vient en butée sur l'extrémité d'une des deux branches de la pince, empêchant ou stoppant de facto la rotation dudit bouchon 31.

De préférence, le bouchon 31 est positionné sur la pince de sorte que les languettes 3201, 3202 viennent en butée contre les extrémités de chacune des deux branches de la pince.

Les différentes parties constituant le bouchon 31, précédemment présenté en référence aux figures 8A et 8B, à l'exception du moyen de prélèvement calibré, sont décrites ci-après.

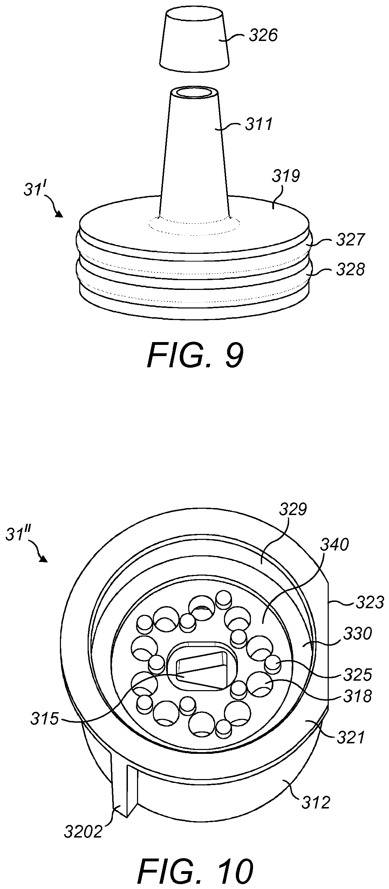

Le bec verseur 31' de ce bouchon 31 est représenté en figure 9. Sur cette dernière, l'on observe, de la partie supérieure vers la partie inférieure, un capuchon amovible 326 de forme complémentaire à celle de l'orifice de forme tubulaire 311. Ce capuchon 326 peut notamment être de forme cylindrique ou de forme conique tronquée.

Le bec verseur 31' comprend également la partie supérieure du corps du bouchon 319 et deux anneaux, 327 et 328, disposés parallèlement l'un à l'autre autour de la partie supérieure du bouchon 319. L'ensemble constitué par les deux anneaux 327 et 328 représente un élément d'emboîtage élastique mâle (communément désigné « élément de clipsage mâle ») adapté pour coopérer avec un élément d'emboîtage élastique femelle (communément désigné « élément de clipsage femelle »). Ce dernier est obtenu par alésage de l'élément central 31", représenté sur la figure 10 et décrit ci-après.

Les figures 8A et 8B représentent également un moyen de positionnement consistant en une zone aplanie 323 du moyen anti-translation 321, permettant de positionner le bouchon par rapport aux extrémités 402 et 403 de chacune des deux branches de la pince 40 et d'éviter ainsi que le moyen d'anti-translation 321 n'entre en contact avec l'appareil de malaxage 71.

La figure 10 permet d'observer précisément la partie supérieure de l'élément central 31", comportant divers supports de filtre(s) 325, 330 adaptés pour recevoir le moyen de filtration représenté par la référence numérique 116 sur la figure 1B. Lesdits supports de filtre(s) comprennent plusieurs ergots 325 positionnés à proximité des ouvertures 318, lesdits ergots 325 s'étendant jusqu'au niveau du support de filtre périphérique 330, consistant en une collerette. Tel qu'indiqué précédemment, l'élément central 31" dispose également d'un élément de clipsage femelle (non représenté sur la figure 10), comprenant deux rainures creusées par alésage dans la paroi latérale interne 329 de l'élément central 31"; ces deux rainures (non représentées) étant adaptées pour recevoir respectivement les anneaux 327 et 328 de l'élément de clipsage mâle du bec verseur 31', et ainsi permettre l'emboîtage élastique du bec verseur 31' dans l'élément central 31".

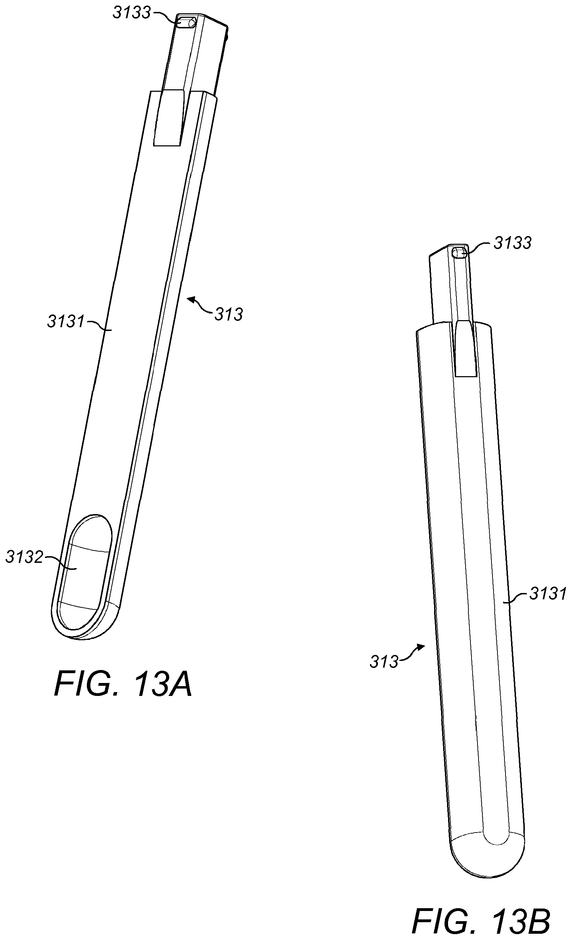

En outre, une ouverture 315 traverse, de part en part, la partie centrale de l'élément central 31". Cette ouverture 315 présente une forme complémentaire à celle du moyen de clipsage mâle 3133 du moyen de prélèvement 313, tel que représenté en figures 13A et 13B. Plus précisément, ladite ouverture 315 est de forme trapézoïdale et est dimensionnée pour que, lorsque le moyen de clipsage mâle 3133 du moyen de prélèvement 313 (cf. figures 13A et 13B infra) est enfiché dans l'ouverture 315, le moyen de prélèvement 313 soit :

(i) immobilisé, et également
(ii) orienté par rapport au moyen anti-rotation 3201, 3202, tel qu'expliqué précédemment, afin d'imposer l'orientation souhaitée à la partie creuse calibrée 3132 (cf. supra).

De surcroît, la complémentarité entre la forme de l'ouverture 315, d'une part, et celle du moyen de clipsage mâle 3133, d'autre part, impose et garantit l'orientation désirée du moyen de prélèvement 313 - et en particulier de sa partie creuse 3132 - lors de l'opération d'assemblage du moyen de prélèvement 313 avec l'élément central 31". En outre, l'emboîtage élastique de la partie 3133 du moyen de prélèvement 313 dans l'ouverture 315 permet de conférer à l'ensemble « élément central 31"-moyen de prélèvement 313 », une rigidité suffisante pour que la tige 3131 du moyen de prélèvement 313 ne se courbe pas durant le prélèvement d'échantillon à matrice hétérogène, évitant, par là-même, les risques de projection d'échantillon à matrice hétérogène.

Les ouvertures 318 telles que représentées sur la figure 10 permettent le passage de la solution de mise en suspension 13 chargée en échantillon à matrice hétérogène du contenant 10 vers la partie intermédiaire 340 de l'élément central 31", contenant le moyen de filtration 116.

Bien que l'assemblage du bec verseur 31' avec l'élément central 31" et de ce dernier avec le moyen de prélèvement soit obtenu par emboîtage élastique de systèmes de clipsage mâles (327, 328 et 3133) et femelles (rainures creusées dans la paroi latérale interne 329 et ouverture 315), l'assemblage de ces différents éléments peut, bien évidemment, être obtenu, de manière alternative, par tout système de connexion/fixation connu de l'homme du métier, tel qu'un système de vissage, collage ou encore soudure (par exemple thermosoudure).

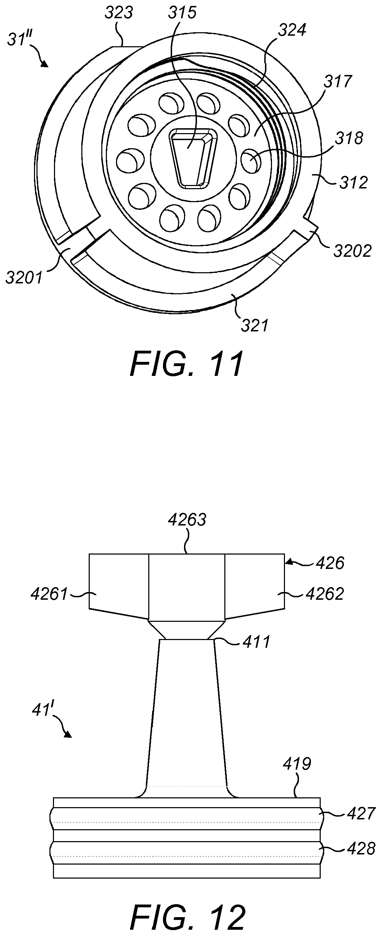

La figure 11 représente une vue de dessous, en légère perspective, de la partie centrale 31" du bouchon 31, sur laquelle l'on observe les ouvertures 318, disposées de manière radiale autour de l'ouverture 315. En jouant sur la disposition relative de l'ouverture trapézoïdale 315 par rapport à celle du moyen anti-rotation 3201, 3202, l'on parvient à orienter la partie creuse 3132 du moyen de prélèvement 313 de sorte que celle-ci soit orientée vers le bas, afin de favoriser la suspension de l'échantillon à matrice hétérogène (tel qu'expliqué précédemment). Sur cette figure 11, l'on note également que la partie interne 317 présente une forme de cône tronqué, permettant de limiter les risques de fuite de la suspension comprenant l'échantillon à matrice hétérogène. Lors du vissage du bouchon sur le contenant, ladite partie en forme de cône tronqué rentre en contact avec le bord du contenant et écrase l'angle du bord du contenant sur une faible surface, assurant ainsi une bonne étanchéité.

Sur cette figure 11, l'on observe également un filet interne 324 obtenu par taraudage, destiné à coopérer avec un filet externe pratiqué sur la partie supérieure du contenant 10, de préférence sur le col du contenant 10, afin de permettre l'assemblage du bouchon 31 et du contenant 10 par vissage. Bien évidemment, d'autres systèmes de fermeture, de préférence hermétiques, pourront être utilisés pour permettre l'assemblage du bouchon et du contenant, par exemple, un système d'emboîtage élastique (clipsage).

La figure 12 représente un deuxième mode de réalisation du bec verseur 41' du bouchon selon l'invention. Ce bec verseur 41' est adapté pour être emboîté par emboîtage élastique à l'intérieur de l'élément central 31" du bouchon 31. Ceci est réalisé en introduisant les anneaux 427 et 428 dans les rainures correspondantes pratiquées dans la paroi latérale interne 329 de l'élément central 31" (cf. figure 10).

Comme montré sur cette figure 12, l'orifice de forme tubulaire 411 de ce bec verseur 41' est solidaire du capuchon sécable et repositionnable 426 (par exemple thermosoudé audit capuchon ou simplement moulé en même temps que le capuchon, à la manière des pipettes de sérum physiologique classiques), de sorte que ledit capuchon sécable et repositionnable 426 empêche, dans cette configuration, toute sortie non contrôlée de liquide via l'orifice de forme tubulaire 411, vers l'extérieur.

En d'autres termes, lorsque ce capuchon sécable et repositionnable 426 est solidaire de l'orifice de forme tubulaire 411, ceci permet d'éviter toute fuite non contrôlée de liquide vers l'extérieur du dispositif selon l'invention. De surcroît, le fait que ce capuchon sécable et repositionnable 426 soit solidaire de l'orifice de forme tubulaire 411 et obstrue ce dernier indique à l'opérateur qu'aucun liquide n'a été versé auparavant via le bec verseur 41'. Le capuchon sécable et repositionnable 427 joue ainsi, indirectement, le rôle de témoin d'inviolabilité.

Le capuchon sécable et repositionnable 426 peut être désolidarisé de l'extrémité de l'orifice de forme tubulaire 411 par l'action d'une force de type traction ou, de préférence, torsion sur ledit capuchon sécable et repositionnable 426, en particulier en exerçant une force de rotation sur une, voire de préférence deux, des ailettes 4261 et 4262 dudit capuchon 426.

L'extrémité du capuchon sécable et repositionnable 426, opposée à l'extrémité en contact avec l'orifice de forme tubulaire 411, comprend une partie creuse 4263 de forme substantiellement cylindrique ou conique tronquée. Cette partie creuse 4263 est de forme complémentaire à celle de l'extrémité de l'orifice de forme tubulaire 411, de sorte que, lorsque le capuchon sécable et repositionnable 426 est désolidarisé de l'orifice de forme tubulaire 411, par exemple en appliquant une traction sur une voire deux des ailettes 4261 et 4262 dudit capuchon 426, ce dernier peut être utilisé, ultérieurement, pour obturer de nouveau l'orifice de forme tubulaire 411 du bec verseur 41'. Cette obturation s'obtient par emboîtement de l'orifice de forme tubulaire 411 dans la partie creuse 4263 du capuchon sécable et repositionnable 426.

Il convient de noter, que selon un mode de réalisation plus simple, le capuchon sécable et repositionnable 426 peut être remplacé par une simple partie sécable de protection, laquelle ne permet pas de reboucher l'orifice de forme tubulaire 411 après désolidarisation de la partie sécable de protection de celui-ci, contrairement au capuchon sécable et repositionnable 426, représenté sur la figure 12.

Les figures 13A et 13B représentent, respectivement, une vue en perspective de trois quart du moyen de prélèvement calibré 313 et une vue de dos de ce même moyen de prélèvement calibré 313. Ces deux figures 13A et 13B nous permettent d'observer le moyen de clipsage mâle 3133, la tige en matériau plein 3131 et la partie creuse calibrée 3132, de forme oblongue (possédant plus précisément une forme dite « en bâtonnet »), ladite partie creuse calibrée 3132 ayant été obtenue par évidement d'une région située à l'extrémité de la tige 3131 opposée à la partie interne du bouchon (non représentée sur les figures 13A et 13B).

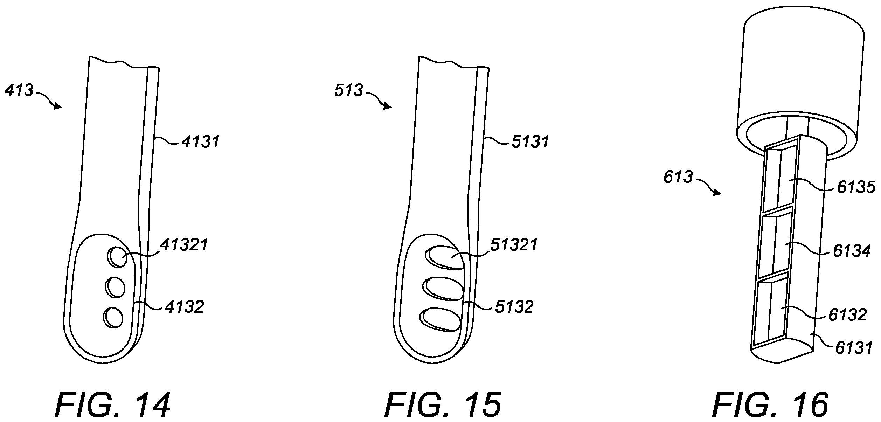

Un deuxième mode de réalisation de la partie creuse 4132 est schématisé en figure 14. Sur cette vue de face, l'on constate que ladite partie creuse calibrée 4132 comprend des ouvertures 41321, destinées à faciliter la mise en suspension de l'échantillon à matrice hétérogène présent au niveau de la partie creuse calibrée 4132. Cet effet technique avantageux est obtenu en permettant à la solution de mise en suspension 13 de passer au travers de la partie creuse calibrée 4132 lors de l'étape de mise en suspension de l'échantillon. Une partie creuse calibrée 4132 selon ce deuxième mode de réalisation s'avère particulièrement adapté pour faciliter la mise en suspension d'échantillons à matrice hétérogène possédant des propriétés adhésives significatives, tels que des selles ayant un type Bristol de 1 ou 2.

La figure 15 illustre un troisième mode de réalisation de la partie creuse calibrée 5132, présentant des ouvertures 51321 de forme ovoïde et de dimensions supérieures à celles des ouvertures 41321 schématisées sur la figure 14. Ces ouvertures 51321 permettent de faciliter davantage la mise en suspension d'un échantillon à matrice hétérogène contenu au sein de la partie creuse calibrée 5132. La partie creuse 5132 selon ce troisième mode de réalisation s'avère particulièrement adaptée pour permettre la mise en suspension d'échantillon ayant des propriétés adhésives plus marquées que celles de l'échantillon à matrice hétérogène devant être prélevées à l'aide ou via la partie creuse calibrée 4132 de la figure 14, telles que des selles ayant un type Bristol de 1-2.

La figure 16 représente un mode de réalisation particulier, dans lequel le moyen de prélèvement 613 comprend, sur une large part de sa tige 6131, trois parties creuses calibrées 6132, 6134 et 6135, de forme carrée ou rectangulaire, possédant chacune un volume prédéterminé pour permettre à chacune de ces trois parties creuses calibrées 6132, 6134 et 6135 de prélever une masse de 1000 mg. Le moyen de prélèvement calibré 613, représenté sur la figure 16, permet donc de prélever une masse totale de 3000 mg d'échantillon à matrice hétérogène. Un moyen de prélèvement calibré 613 de ce type peut s'avérer particulièrement important pour récupérer *in fine* un filtrat contenant une concentration de matière biologique et/ou d'information biologique suffisante afin de permettre leur analyse, notamment dans le cas où la matière biologique et/ou l'information biologique recherchée(s) est(sont) connue(s) pour être présente(s) en très petite quantité dans la matière à matrice hétérogène à partir de laquelle l'échantillon est prélevé. Ce moyen de prélèvement 613 peut en outre être utilisé, de manière avantageuse, dans des applications vétérinaires, en augmentant au besoin le volume défini par chacune des parties creuses 6132, 6134 et 6135.

Selon un mode de réalisation particulier, la tige 6131 du moyen de prélèvement 613 présente, sur sa face opposée à celle comprenant les trois parties creuses calibrées 6132, 6134 et 6135, trois parties creuses calibrées additionnelles. De préférence, ces trois parties creuses calibrées additionnelles permettent chacune de prélever une masse d'échantillon à matrice hétérogène d'environ 1000 mg. Ainsi, ce moyen de prélèvement adapté comprenant 6 parties creuses calibrées permet de prélever une masse totale d'échantillon à matrice hétérogène d'environ 6000 mg d'échantillon à matrice hétérogène.

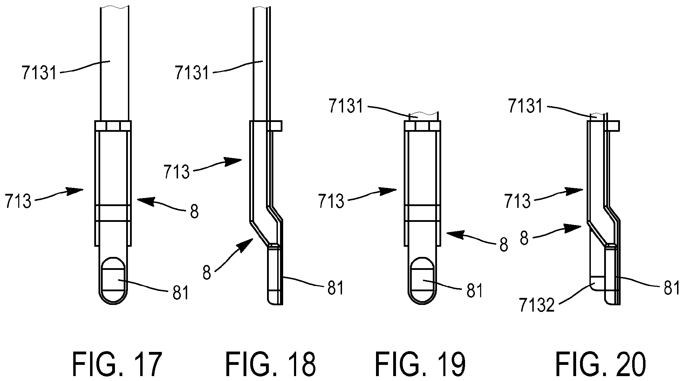

Comme représenté sur les figures 17 à 20, selon un autre mode de réalisation préféré de l'invention, la tige 7131 du moyen de prélèvement calibré 713 est connectée à une extension coulissante 8. Cette extension coulissante 8 permet de prolonger la longueur de la tige 7131 dudit moyen de prélèvement calibré 713 vers une première longueur de tige. Cette extension coulissante 8 est positionnée par translation sur le moyen de prélèvement calibré 713, lequel comprend déjà une partie creuse calibrée 7132 (que l'on peut apercevoir sur la figure 20). Lorsque l'extension coulissante 8 est en position "sortie", à savoir permettant de conférer la susdite première longueur de tige (cf. figures 17 et 18), l'opérateur peut plus facilement procéder au prélèvement d'un échantillon à matrice hétérogène, par exemple un échantillon de selles, au fond d'un tube étroit, au moyen d'au moins une partie creuse calibrée 81, positionnée au niveau de l'extrémité de l'extension coulissante 8 la plus éloignée de la partie interne du bouchon, en évitant tout contact entre le bouchon et les bords dudit tube.

Après prélèvement de l'échantillon à matrice hétérogène, et tel qu'indiqué précédemment, le moyen de prélèvement calibré 713 et le contenant sont assemblés afin de permettre la mise en suspension de l'échantillon à matrice hétérogène et, ce faisant, poursuivre le procédé d'obtention de matière biologique et/ou d'information selon l'invention. Lors de cette opération d'assemblage, l'extension coulissante 8 en position « sortie » (première longueur de tige ; cf. figures 17 et 18) entre en butée avec le fond du contenant et, sous l'effet de la résistance opposée par le fond du contenant, l'extension coulissante 8 coulisse le long de la tige 7131, en direction de la partie interne du bouchon, vers sa position « rentrée » (deuxième longueur de tige ; cf. figures 19 et 20), afin de permettre l'assemblage du bouchon et du contenant, par exemple en vissant le bouchon sur un pas de vis positionné sur le col du contenant, tel qu'indiqué précédemment.

Selon un mode de réalisation de l'invention, en vue de procéder au prélèvement d'échantillon à matrice hétérogène, l'opérateur désassemble le bouchon et le contenant, puis fait coulisser l'extension coulissante 8 connectée à la tige 7131 le long de cette dernière, de la position « rentrée » (deuxième position ; cf. figures 19 et 20) vers la position « sortie » (première position ; cf. figures 17 et 18). Selon une variante, afin d'éviter toute manipulation du moyen de prélèvement calibré 713 préalablement à l'étape de prélèvement (susceptible de générer une contamination microbienne croisée), le bouchon peut être fourni dans un emballage stérile, l'extension coulissante 8 d'ores et déjà en position « sortie » (première longueur de tige ; cf. figures 17 et 18). Ainsi, le prélèvement d'échantillon à matrice hétérogène peut être directement effectué sans que l'opérateur n'ait besoin de toucher l'extension coulissante 8.

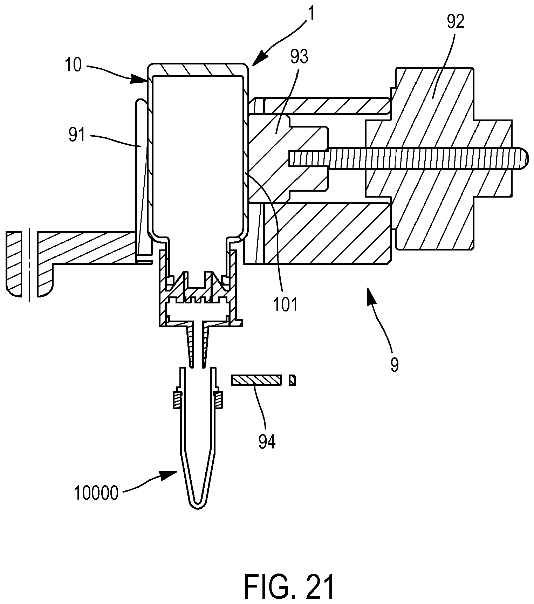

Tel qu'indiqué précédemment, la figure 21 est une vue en coupe d'un automate de filtration 9 (ou dispositif automatique de filtration) selon l'invention. Cet automate de filtration permet d'automatiser l'étape de filtration du contenu (à savoir de la suspension contenant l'échantillon à matrice hétérogène d'intérêt) présent au sein du dispositif d'obtention de matière biologique et/ou d'information biologique selon l'invention.

L'opérateur positionne le dispositif d'obtention de matière biologique et/ou d'information biologique selon l'invention 1 dans un emplacement 91 adapté pour recevoir et maintenir le dispositif 1 lors de l'opération de filtration.

L'opérateur met ensuite en route un moteur 92 (par exemple en appuyant sur un bouton poussoir), ce qui a pour effet d'induire le passage d'un organe de pression 93 d'une position initiale (« position de repos » - non représentée sur la figure 21) vers une position « de pression » (visible sur la figure 21), dans laquelle ledit organe de pression 93 exerce une pression contre une zone en matière souple 101 du contenant 10.

La pression exercée par l'organe de pression 93 sur ladite zone en matière souple 101 du contenant 10 est maintenue grâce au moteur 92 durant un laps de temps nécessaire à la collecte d'un volume souhaité de filtrat au sein d'un récipient collecteur 10000 (par exemple au sein d'un tube eppendorf®).

L'automate de filtration 9 comprend, de manière particulièrement avantageuse, un détecteur optique de niveau 94 (également dénommé « barrière optique »). Ce détecteur optique de niveau 94 permet de stopper l'étape de filtration lorsque le niveau souhaité de filtrat est atteint au sein du récipient collecteur 10000. Plus précisément, ce détecteur optique fonctionne en induisant le passage de l'organe de pression 93 de la position de pression vers la position de repos (sous l'action du moteur 92 ou, plus simplement, par arrêt de celui-ci), à savoir stoppe la pression exercée par l'organe de pression 93 sur la zone en matière souple 101 du contenant 10 lorsque le détecteur optique de niveau détecte, via un capteur optique, que le niveau souhaité de filtrat est atteint au sein du récipient collecteur 10000, mettant ainsi fin à l'étape de filtration.

Tel qu'expliqué précédemment, le fait que l'organe de pression 93 cesse d'exercer une pression contre ladite zone en matière souple 101 du dispositif 1 a pour effet de mettre fin à la surpression précédemment générée à l'intérieur du contenant 10, ce qui conduit à l'arrêt de l'étape de filtration lorsque le niveau souhaité de filtrat est atteint au sein du récipient collecteur 10000.

Comme mentionné supra, l'automate de filtration 9, pourvu d'un détecteur optique de niveau 94, s'avère particulièrement avantageux, dans la mesure où il permet de collecter le même volume de filtrat, et ce quel que soit le type de selles, assurant répétabilité et robustesse au procédé d'obtention de matière biologique et/ou d'information biologique mettant en œuvre le dispositif 1 selon l'invention. Ceci est d'autant plus vrai que, comme expliqué précédemment, la partie creuse calibrée du dispositif 1 selon l'invention permet de prélever une masse donnée/prédéfinie d'échantillon à matrice hétérogène (sans avoir à effectuer des opérations de pesée), ladite masse étant constante ou quasi-constante à chaque opération de prélèvement d'échantillon à matrice hétérogène. En d'autres termes, ladite partie creuse calibrée et l'automate de filtration 9 selon l'invention, pourvu d'un détecteur optique de niveau 94, agissent en synergie afin de garantir une répétabilité et une robustesse optimale au procédé d'obtention de matière biologique et/ou d'information biologique mettant en œuvre le dispositif 1 selon l'invention.

Même si, dans un souci de clarté, un seul emplacement 91 est représenté sur l'automate de filtration 9 objet de la figure 21, de façon optimale, cet automate de filtration 9 comprend jusqu'à huit voire dix emplacements 91 différents, ce qui permet de filtrer simultanément jusqu'à huit voire dix dispositifs 1, et ce avec une répétabilité et une robustesse optimales, tel qu'expliqué supra.

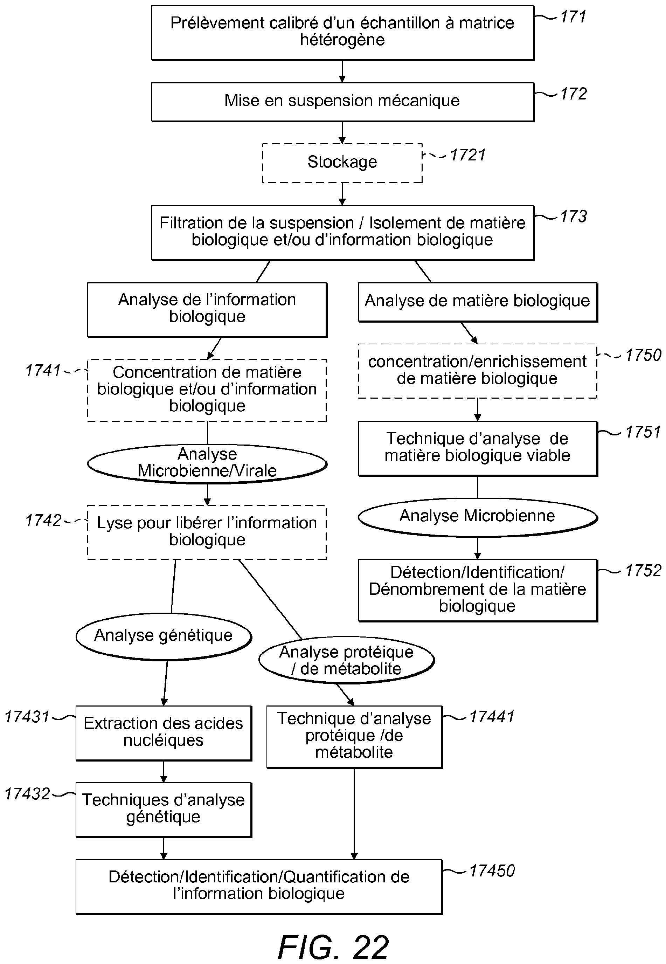

La figure 22 est un diagramme représentant:

(i) les différentes étapes essentielles (en trait plein) et optionnelles (en pointillés) du procédé d'obtention de matière biologique et/ou d'information biologique selon l'invention

(ii) les différentes étapes nécessaires (en trait plein) et optionnelles (en pointillés) des différentes voies d'analyse subséquentes, permettant l'identification et/ou la détection et/ou la quantification de ladite matière biologique et/ou ladite information biologique.

Les étapes 171, 172 et 173, représentées sur la figure 22, sont décrites en lien avec le dispositif 1, représenté sur les figures 1A et 1B.

Afin d'effectuer l'étape de prélèvement 171, l'opérateur saisit manuellement le bouchon, de préférence par le corps de bouchon, et remplit le volume défini par la partie creuse calibrée avec l'échantillon à matrice hétérogène (par exemple un échantillon de selles). Le bouchon et le contenant, préalablement remplis avec une solution de mise en suspension, sont assemblés, par exemple par vissage du bouchon sur le col du contenant, de sorte que l'échantillon à matrice hétérogène contenu dans la partie creuse soit en contact avec la solution de mise en suspension.

Il convient de noter que le contenant peut contenir, outre la solution de mise en suspension, un ou plusieurs moyens de mise en suspension, par exemple 30 billes en verre d'un diamètre de 3 mm chacune.

L'étape de mise en suspension 172 peut être réalisée en agitant le dispositif afin de permettre la suspension de l'échantillon à matrice hétérogène contenu dans la partie creuse calibrée dans la solution de mise en suspension. Afin de faciliter et/ou d'accélérer cette étape de mise en suspension mécanique 172, le dispositif peut être connecté à un moyen de malaxage, tel qu'un vortex, comme représenté sur la figure 6. Avantageusement le dispositif est connecté audit moyen de malaxage par un organe de maintien coopérant étroitement avec la partie rigide du bouchon, afin d'assurer un maintien efficace du dispositif durant l'étape de mise en suspension mécanique 172, consistant, en l'espèce, en une étape de malaxage.

Une fois que l'opérateur estime que l'échantillon à matrice hétérogène a été correctement mis en suspension au sein de la solution de mise en suspension, l'étape de mise en suspension 172 est achevée. A ce moment-là, le dispositif contenant la suspension d'échantillon à matrice hétérogène peut éventuellement être stocké, à l'étape 1721, de préférence après ajout d'au moins un moyen de conservation et/ou par congélation. Alternativement, le dispositif peut également être incubé en température, par exemple à 37°C, pour une durée de quelques heures à plusieurs jours afin d'enrichir la suspension de certains micro-organismes présents dans l'échantillon. Cette étape d'incubation peut notamment être réalisée en présence d'un moyen de culture sélectif contenu dans le dispositif selon l'invention. La durée maximale de stockage dépend bien évidemment du moyen de conservation utilisé, le cas échéant, et de la nature de la matière biologique et/ou de l'information biologique à analyser. Tel qu'indiqué précédemment, cette étape de stockage peut s'avérer particulièrement intéressante dans le cadre d'opérations de prélèvement d'échantillons à matrice hétérogène « hors laboratoire ». En effet, le dispositif comprenant éventuellement un moyen de conservation peut, après que l'orifice de forme tubulaire a été obturé par un capuchon, être envoyé à un laboratoire d'analyse en vue d'effectuer toutes les analyses souhaitées de la matière biologique et/ou de l'information biologique susceptible(s) d'être contenue(s) dans l'échantillon à matrice hétérogène prélevé à l'étape 171.

Alternativement, l'opérateur peut directement procéder à l'étape de filtration 173. Afin de réaliser cette étape de filtration 173, le moyen d'obturation amovible est ôté de l'orifice de forme tubulaire, puis le dispositif est ensuite retourné et au moins une pression est appliquée sur au moins une zone de la paroi souple du contenant, afin de créer une surpression dans ce contenant et ainsi forcer le passage de la solution de mise en suspension chargée en échantillon à matière hétérogène au travers du moyen de filtration du bouchon, via les ouvertures. Ainsi, la solution de mise en suspension chargée en échantillon à matière hétérogène qui s'écoule le long de l'orifice de forme tubulaire a obligatoirement été filtrée par le moyen de filtration 116 positionné dans le corps du bouchon. A l'issue de cette étape de filtration 173, le filtrat contenant ladite matière biologique et/ou information biologique est versé via l'orifice de forme tubulaire dans un tube collecteur, par exemple un tube Eppendorf. Une fois ladite matière biologique et/ou information biologique isolée(s) dans le tube collecteur, l'opérateur peut procéder à différentes analyses biologiques, en fonction de la nature de la matière biologique et/ou information biologique recherchée(s).

Lorsque l'on souhaite analyser la matière biologique viable, des techniques de microbiologie classiques 1751 peuvent être réalisées à ces fins, telles que l'ensemencement et la mise en culture de ladite matière biologique viable sur des milieux réactionnels solides, liquides ou semi-solides, sélectifs ou non sélectifs, comprenant, de préférence, un milieu de culture. L'expression métabolique de cette matière biologique viable peut également être évaluée via l'utilisation de tests enzymatiques (par exemple galeries API, plaque MALDI-TOF, test par flux latéral). Ces techniques de microbiologie permettent de détecter et/ou identifier et/ou dénombrer, à l'étape 1752, la matière biologique viable recherchée. Toutefois, en fonction des techniques d'analyse utilisées, du type de matière biologique viable d'intérêt, une étape additionnelle d'enrichissement peut s'avérer nécessaire afin de favoriser la croissance de ladite matière biologique et ainsi augmenter sa concentration dans le milieu réactionnel.

Bien évidemment, ladite matière biologique viable, tout comme l'information biologique au sens large, peut également faire l'objet d'analyses génétiques, protéiques et/ou métaboliques. A cet effet, le lecteur se référera à l'analyse d'information biologique, laquelle est décrite ci-après.

Postérieurement à l'étape de filtration 173, lorsque l'opérateur souhaite analyser l'information biologique contenue dans le filtrat, et comprenant notamment des métabolites, des protéines ou encore des acides nucléiques, des techniques spécifiques de détection et/ou d'identification et/ou de quantification sont réalisées. De manière particulièrement avantageuse, une étape de concentration de ladite information biologique 1741 contenue dans le filtrat est effectuée par centrifugation à 6000-12000g ou par floculation, afin de concentrer l'information biologique d'intérêt et de diminuer la quantité d'éléments non ciblés (tels que des inhibiteurs) présents dans la suspension. Le cas échéant, une étape de lyse 1742 est effectuée, lorsque l'information biologique d'intérêt est contenue dans ladite matière biologique, en particulier lorsque ladite matière biologique est une matière biologique autoreproductible, afin de rendre accessible l'information biologique au(x) moyen(s) d'analyse(s).

Selon une variante, l'étape de lyse 1742 peut être réalisée avant l'étape de concentration 1741.

Les étapes subséquentes dépendent de la nature de l'information biologique recherchée et des techniques d'analyses employées, techniques d'analyses génétiques, protéiques ou métaboliques. Lorsque l'opérateur souhaite analyser l'information biologique d'intérêt par le biais d'une technique d'analyse génétique (analyse d'acide(s) nucléique(s)), une étape 17431 d'extraction des acides nucléiques est réalisée en mettant en œuvre tout protocole d'extraction d'acides nucléiques adapté. Les acides nucléiques obtenus à l'issue de l'étape d'extraction 17431 sont détectés et/ou identifiés et/ou quantifiés par toute méthode d'analyse génétique appropriée 17432, par exemple par PCR.

L'analyse protéique et/ou métabolique 17441 fait intervenir, quant à elle, des techniques d'analyse adaptées, telles que des essais immunologiques (immunodosages) ou encore des essais enzymatiques (liste non limitative), lesquels permettent, à l'étape 17450, de détecter et/ou identifier et/ou quantifier les protéines et/ou les métabolites d'intérêt, initialement présents dans l'échantillon à matrice hétérogène.

Les exemples ci-après permettront de mieux appréhender la présente invention. Toutefois, ces exemples ne sont donnés qu'à titre illustratif et ne doivent en aucun cas être regardés comme limitant la portée de ladite invention d'une quelconque manière.

**Exemples**

Exemple 1 - Assemblage d'un dispositif selon l'invention

Aux fins de l'exemple 1, le dispositif d'obtention de matière biologique et/ou d'information biologique à partir d'un échantillon à matrice hétérogène est assemblé de la façon suivante :

- insertion du premier filtre 1161 (Filtrona référence : BNW440148) dans la partie centrale de l'élément central 31" du bouchon 31,
- insertion d'un second filtre 1162 (Pall Pad référence : 66025) en superposition du premier filtre 1161,
- mise en place du bec verseur 41' par emboîtage élastique des systèmes de clipsage mâles 427 et 428 et femelles (rainures creusées dans la paroi latérale interne 329 de l'élément central 31"),
- clipsage du moyen de prélèvement calibré 313, présentant une partie creuse calibrée 3132 (volume : 300 $\mu$L), dans l'ouverture 315 de l'élément central 31",
- ajout de 30 billes de verre d'un diamètre de 3 mm dans le contenant 10,
- ajout de 5 mL de solution de mise en suspension (en l'espèce tampon de mise en suspension de type tampon TE) dans le contenant 10, puis
- vissage du bouchon comprenant le bec verseur 41' et le moyen de prélèvement calibré 313 sur le col du contenant 10.

Exemple 2 - Procédé d'obtention de matière biologique et/ou d'information biologique mettant en œuvre le dispositif de l'exemple 1

Le procédé d'obtention de matière biologique et/ou d'information biologique à partir d'un échantillon à matrice hétérogène selon la présente invention, mettant en œuvre le dispositif décrit à l'exemple 1 comprend les étapes suivantes :

1) Prélèvement de l'échantillon

De manière plus précise, l'utilisateur ouvre, dans un premier temps, le pot contenant les selles à analyser, et prélève un échantillon à l'aide du moyen de prélèvement calibré 313, de sorte que la partie creuse calibrée 3132 du moyen de prélèvement calibré 313 soit remplie de l'échantillon d'intérêt. Afin de permettre une calibration optimale, le moyen de prélèvement calibré 313 est ensuite « raclé » sur le rebord du pot susvisé afin d'éliminer le surplus éventuel de selles présent sur le moyen de prélèvement calibré 313 et/ou débordant de la partie creuse calibrée 3132.

2) Mise en suspension de l'échantillon

Dans un deuxième temps, l'utilisateur introduit le moyen de prélèvement calibré 313 dans le contenant 10, et visse fermement le bouchon 31 avant de fixer le dispositif selon l'invention sur un appareil de malaxage 71 (vortex Genie II; Scientific Industries, Inc.). Plus précisément, comme représenté en figure 6, le bouchon du dispositif selon l'invention est maintenu par l'intermédiaire d'une pince 40 connectée à un « plateau vortex », lui-même disposé sur l'appareil de malaxage 71 (« vortex »). Le dispositif ainsi fixé est malaxé (« vortexé ») jusqu'à obtenir la mise en suspension complète de l'échantillon, à savoir durant un laps de temps généralement compris entre environ 30 secondes et environ 2 minutes suivant le type Bristol. Bien évidemment, la durée optimale de malaxage (« vortexage ») de l'échantillon pourra aisément être déterminée par l'utilisateur sur la base de ses connaissances générales et, le cas échéant, de tests de routine.

3) Filtration de l'échantillon mis en suspension à l'étape 2)

Dans un troisième temps, une fois l'étape de mise en suspension achevée, l'utilisateur retire le dispositif selon l'invention du « plateau vortex », puis enlève le capuchon 426 en appliquant une traction sur les ailettes 4261 et 4262, retourne le dispositif au-dessus d'un tube collecteur (Eppendorf 2 mL), puis exerce une pression avec ses doigts sur les parois souple 101 du contenant 10 jusqu'à recueillir le volume souhaité de filtrat, à savoir environ 2 mL. Le filtrat ainsi obtenu est prêt pour être analysé.

Exemple 3 - Efficacité de la calibration du moyen de prélèvement 313 sur des échantillons de selles de différents types Bristol.

Le prélèvement est effectué à partir d'échantillons de selles de donneurs sains, stockées à une température de -80°C. Les selles sont préalablement décongelées.

Les prélèvements sont effectués en mettant en œuvre l'étape 1 du procédé de l'exemple 2.

Afin de déterminer précisément la masse de selles prélevée dans la partie creuse calibrée 3132 du moyen de prélèvement 313, le bouchon est pesé avant (tare) et après l'opération de prélèvement.

Les résultats obtenus sont présentés dans le tableau 1 ci-dessous :

| Bristol | Bristol 2 | Bristol 3 | Bristol 3 | Bristol 4 | Bristol 5 | Bristol 6 |
|---|---|---|---|---|---|---|
| Poids selles par prélèvement (mg) | 189 | 201 | 214 | 217 | 217 | 191 |
| | 199 | 200 | 215 | 271 | 209 | 191 |
| | 143 | | 259 | 223 | | 211 |
| | 206 | | 242 | 216 | | 236 |
| | | | 207 | 187 | | 220 |
| | | | 216 | 258 | | 234 |
| | | | 220 | 236 | | |
| | | | 254 | 224 | | |
| | | | 209 | 204 | | |
| | | | 192 | 244 | | |
| | | | 192 | 191 | | |
| | | | 198 | 200 | | |
| Moyenne | 184 | 201 | 218 | 223 | 213 | 214 |
| CV (%) | 15 | 0.4 | 10 | 12 | 3 | 9 |

Tableau 1

L'exemple 3 permet de calculer la variabilité de prélèvement (CV), correspondant à l'ensemble des étapes mises en œuvre pour réaliser le prélèvement.

Le CV est un pourcentage calculé par le rapport de l'écart-type sur la moyenne, en appliquant la formule suivante :

$$\sqrt{\frac{\sum (x - \bar{x})^2}{n}}$$

où $\bar{x}$ est la moyenne (nombre 1, nombre 2...) de l'échantillon et n est la taille de l'échantillon.

La partie creuse calibrée 3132 du moyen de prélèvement 313 permet de collecter une masse donnée/prédéterminée de selles de manière aisée, efficace et reproductible (robustesse du procédé d'obtention de matière biologique et/ou d'information biologique mettant en œuvre le dispositif selon l'invention) et ce pour différents types Bristol. Les variations mineures de masse constatées en répétabilité sont considérées acceptables en ce qu'elles n'affectent pas, de manière significative, la quantité de matière biologique et/ou d'information biologique obtenue par la mise en œuvre du dispositif.

Exemple 4 - Test de linéarité de détection des acides nucléiques de différents types de micro-organismes en utilisant le procédé d'analyse d'information biologique selon l'invention.

Afin de contrôler l'efficacité du procédé d'analyse d'information biologique (en l'espèce d'ADN) à partir d'un échantillon à matrice hétérogène, objet de la présente invention, les trois types de micro-organismes suivants ont été inoculés dans la solution de mise en suspension contenue au sein du dispositif selon l'invention:

- *Klebsiella pneumoniae* résistant aux carbapénèmes (KPC),
- *Staphylococcus aureus* résistant à la méthicilline (MRSA),
- *Schizosaccharomyces pombe* (*S. pombe*).

Ces trois types de micro-organismes ont été choisis d'après leur taille et leurs caractéristiques, KPC étant un bacille Gram négatif de 5 $\mu$m de long résistant aux carbapénèmes, MRSA étant un coque Gram positif résistant à la méticilline de 1 $\mu$m de diamètre et S. *pombe* étant une levure de 10 $\mu$m de long et de forme cylindrique.

L'inoculation de la solution de mise en suspension se prépare de la manière suivante:

- MRSA et KPC :

MRSA et KPC se cultivent sur gélose TSA (Gélose Trypticase Soja) pendant 1 nuit à 37°C. Une solution de 7 McFarland correspondant à 2.10$^9$ CFU/mL (utilisation d'un densitomètre) est préparée et diluée en cascade (au 1/10$^{ème}$) jusqu'à la dilution 10$^3$ CFU/mL. 100 $\mu$L des dilutions concernées sont utilisés pour inoculer la solution de mise en suspension avant l'ajout de l'échantillon de selles. 100 $\mu$L de la dilution à 10$^3$ CFU/mL sont ensuite déposés sur boîtes COS (Référence : 43041, bioMérieux) (Gélose Columbia + 5% de sang de mouton) en triplicata afin de vérifier la concentration de l'inoculum. Les colonies sont comptées après 24h d'incubation (37°C).

- *S. pombe* :

*S. pombe* se cultive pendant 2 à 3 jours sur gélose SDC (Gélose Sabouraud glucosée ; référence : 43555, bioMérieux) à 30°C, puis 1 nuit dans un bouillon Sabouraud (30°C) (Référence : 42108, bioMérieux), la densité optique est mesurée au spectrophotomètre et le bouillon est dilué en cascade jusqu'à la dilution 10$^5$ CFU/mL. 100 $\mu$L des dilutions concernées sont utilisés pour inoculer la solution de mise en suspension avant l'ajout de l'échantillon de selles. Un comptage direct des levures au microscope à l'aide d'une cellule de Kova est ensuite réalisé à partir de la dilution à 10$^6$ CFU/mL afin de vérifier la concentration des inocula.

Chaque solution de mise en suspension de chaque dispositif est respectivement inoculée avec les quantités de MRSA et de KPC suivantes : 0, 10$^5$, 10$^7$, 10$^8$ et 2.10$^9$ CFU. Pour S. *pombe,* les quantités inoculées sont : 0, 10$^5$, 10$^6$ et 10$^7$ CFU.

Trois contrôles sont systématiquement réalisés lors des tests:

- Contrôle négatif n°1 : Dispositif selon l'invention, comprenant une solution de mise en suspension (Tampon TE) non inoculée et un échantillon de selles analysé au préalable afin de s'assurer de l'absence de MRSA, de KPC et de S. *pombe* dans ces selles,
- Contrôle négatif n°2 : Dispositif selon l'invention comprenant une solution de mise en suspension (Tampon TE) non inoculée, sans échantillon de selles, afin de vérifier s'il n'y a pas eu de contaminations lors des manipulations,
- Contrôle positif : Dispositif selon l'invention comprenant une solution de mise en suspension (Tampon TE) inoculée avec l'ensemble des trois micro-organismes testés à une concentration connue, sans échantillon de selles, afin de déterminer l'impact des selles sur la détection par PCR quantitative des micro-organismes.

Dans le contenant 10 ont été ajoutés : 5 mL de tampon TE et 30 billes en verre d'un diamètre de 3 mm. L'inoculation de la solution de mise en suspension est alors effectuée par ajout de 100 $\mu$L des suspensions préalablement préparées. Après inoculation, le dispositif est utilisé pour réaliser les étapes de prélèvement, mise en suspension et filtration comme décrit à l'exemple 2.

L'étape de prélèvement 1) est effectuée à partir d'un échantillon de selles de donneurs sains, de selles stockées en pot et congelées à -80°C. Les selles connues pour contenir naturellement KPC, MRSA ou S. *pombe* sont écartées.

Le filtrat recueilli à l'issue de l'étape de filtration 3) (cf. exemple 2) est centrifugé 3 minutes à 12000g afin de concentrer les micro-organismes. Le surnageant est jeté et le culot est resuspendu dans 600 $\mu$L de tampon TE en vortexant vigoureusement.

Les 600 $\mu$L ainsi obtenus sont ensuite versés dans un tube de lyse (Eppendorf 1,5 mL) pré-rempli d'un mélange de billes consistant en 150 $\mu$g de billes zirconium de 0,1 mm de diamètre et de 600 $\mu$g de billes en verre de 1 mm de diamètre. Par la suite, le tube Eppendorf est disposé sur un « plateau vortex » (plateau horizontal 24 positions), lui-même placé sur un vortex Genie 2 (Scientific Industries, Inc.), pendant 20 minutes et à puissance maximale, afin de lyser les micro-organismes contenus dans le tube Eppendorf.

La solution ainsi lysée est récupérée par pipetage et les billes sont lavées avec 200 $\mu$L de tampon TE. Le tampon TE ayant servi au lavage des billes est récupéré et ajouté à la solution précédemment récupérée par pipetage.

L'ensemble du volume récupéré est ensuite séparé en deux aliquotes, chaque aliquote étant introduite dans un tube contenant 2 mL de tampon de lyse easyMAG® (Référence : 280134, bioMérieux) et 140 $\mu$L de silice EasyMAG®

(Référence: 280133, bioMérieux). Chaque mélange est ensuite disposé dans un puits d'une navette EasyMAG® (bio-Mérieux). Le protocole spécifique « B » est lancé, incluant une lyse « off-board » et une élution dans un volume de 50 μL. Les 2x50 μL d'éluat provenant d'un même dispositif sont mélangés, après extraction, dans un même tube.

Les réactifs et consommables de l'easyMAG® sont listés ci-après :

Référence: 280134 Tampon de lyse NucliSENS® easyMAG® (4 x 1000 mL/bouteille) Référence: 280130 Tampon d'extraction 1 NucliSENS® easyMAG® (4 x 1000 mL/bouteille)
Référence: 280131 Tampon d'extraction 2 NucliSENS® easyMAG® (4 x 1000 mL/bouteille)
Référence: 280132 Tampon d'extraction 3 NucliSENS® easyMAG® (4 x 1000 mL/bouteille)
Référence: 280133 Silice magnétique NucliSENS® easyMAG® (48 x 0,6 mL/flacon) Référence: 280135 Consommables NucliSENS® easyMAG®
Référence: 280146 Embouts pour multipette

Après extraction, 5 μL d'éluats sont utilisés pour effectuer une analyse par PCR ciblant chacun des trois micro-organismes inoculés. Les résultats sont exprimés en Cq (cycle de quantification), lequel est directement lié à la concentration d'ADN présent dans le tube d'amplification en fonction du log de la concentration en micro-organismes inoculés.

Les amorces et les sondes utilisées pour l'analyse moléculaire sont spécifiques des gènes KPC [gène codant pour les carbapénèmases) chez *K. pneumoniae,* des gènes SCCmec (gène de résistance à la méticilline) chez MRSA et des gènes SPBPJ4664.02 (glycoprotéine) chez S. *pombe.*

Pour chacune des séquences d'intérêt, les mix PCR sont préparés conformément aux indications données au sein des tableaux 2-4 infra :

Tableau 2 - Mix des réactifs PCR pour l'analyse des KPC

| Réactifs | Concentration finale |
|---|---|
| Eau Acros | - |
| Tampon pH 8,6 (X) | 1 |
| Solution MgCl$_2$ (mM) | 3.5 |
| dNTP (mM) | 0.2 |
| ASB (μg/μL) | 0.5 |
| Amorce anti-sens (μM) | 0.2 |
| Amorce sens (μM) | 0.2 |
| Sonde (μM) | 0.1 |
| ADN Polymérase (Fast Start) (U/μL) | 0.08 |

Tableau 3 - Mix des réactifs PCR pour l'analyse des *S. pombe*

| Réactifs | Concentration finale |
|---|---|
| Eau Acros | - |
| Tampon pH 8,6 (X) | 1 |
| Solution MgCl$_2$ (mM) | 5 |
| dNTP (mM) | 0.2 |
| BSA ASB (μg/μL) | 0.5 |
| Amorce anti-sens (μM) | 0.65 |
| Amorce sens (μM) | 0.65 |
| Sonde (μM) | 0.17 |
| ADN Polymérase (Fast Start) (U/μL) | 0.08 |

Tableau 4 - Mix des réactifs PCR pour l'analyse des MRSA

| Réactifs | Concentration finale |
|---|---|
| Eau Acros | - |
| Tampon pH 8,6 (X) | 1 |
| Solution MgCl$_2$ (mM) | 5 |
| dNTP (mM) | 0.2 |
| ASB ($\mu$g/$\mu$L) | 0.5 |
| Amorce anti-sens ($\mu$M) | 0.2 |
| Amorce sens ($\mu$M) | 0.2 |
| Sonde ($\mu$M) | 0.1 |
| ADN Polymérase (Fast Start) (U/$\mu$L) | 0.144 |

Pour chaque réaction PCR, les mix de réactifs PCR sont préparés dans un volume final de 20 $\mu$L, auxquels sont additionnés 5 $\mu$L des éluats obtenus pour chaque échantillon traité. Les mix sont ensuite amplifiés à l'aide d'un thermocycleur, selon les cycles d'amplification présentés dans le tableau 5 ci-dessous :

Tableau 5

| | Activation Enzymatique (Fast Start) | Dénaturation | Hybridation/ Elongation |
|---|---|---|---|
| Température (°C) | 95 | 95 | 65°C |
| Temps | 5 min | 15 sec | 45 sec |
| Cycle(s) | 1 | 50 | |

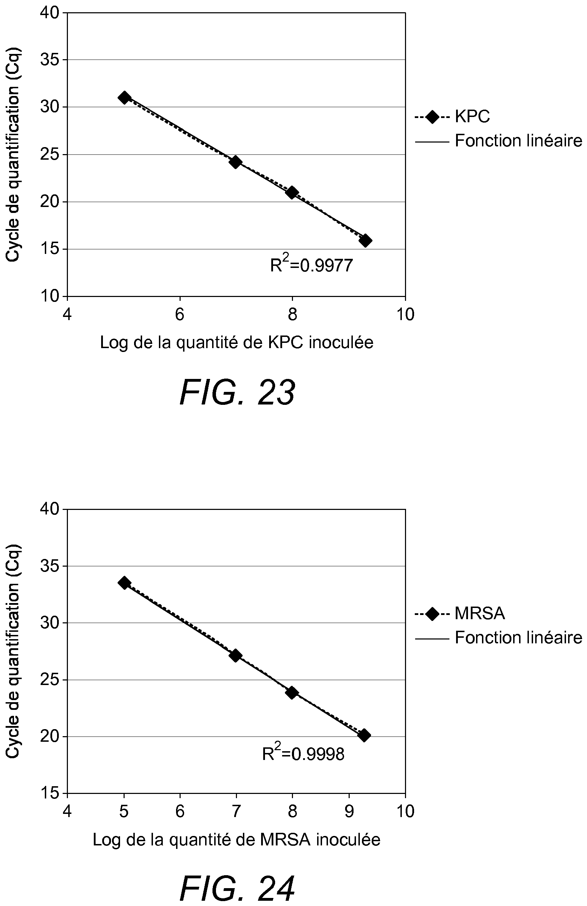

Les résultats obtenus (présentés en figures 23, 24 et 25) montrent une relation linéaire entre la quantité de micro-organismes inoculés préalablement dans les solutions de mise en suspension et la quantité d'ADN amplifiée en PCR retrouvée dans l'éluat après extraction. Par conséquent, cela démontre que le procédé d'analyse d'information biologique selon l'invention permet de détecter de façon « LOG-linéaire » les micro-organismes présents dans les échantillons de selles, et ce sur toute la gamme de quantités testées.

En conclusion, au vu des résultats ainsi obtenus, il apparaît que le procédé d'analyse d'information biologique selon l'invention est efficace pour isoler et identifier les micro-organismes présents dans des échantillons de selles (y compris les levures), aussi bien quantitativement que qualitativement.

Exemple 5 - Comparaison du procédé d'analyse d'information biologique selon l'invention et du protocole mettant en œuvre le kit QIAamp® DNA stool (QiaGen : Référence : 515041

La préparation des inocula a été réalisée en suivant la méthodologie exposée dans l'exemple 4, à partir d'une solution à 0,5 McF correspondant à 10$^8$ CFU/mL. L'inoculation de la solution de mise en suspension est réalisée avec 10$^8$ CFU de MRSA, S. *pombe* et KPC. Le contrôle des inocula est réalisé comme dans l'exemple 4. Il en va de même pour les contrôles négatifs n° 1 et n° 2 et pour le contrôle positif.

L'obtention et la quantification de l'information biologique sont effectuées en mettant en œuvre le procédé d'analyse d'information biologique (en l'espèce d'ADN) selon l'invention, décrit à l'exemple 4.

L'obtention d'information biologique avec le kit QIAamp® DNA stool est réalisée en suivant le protocole expérimental fourni par le fournisseur (QiaGen). Néanmoins, considérant la présente application métagénomique, une étape de lyse mécanique a été rajoutée après ajout du tampon ASL et avant incubation à 95°C pendant 10 minutes, afin d'obtenir un meilleur rendement de lyse des micro-organismes. Ceci relève des connaissances générales de l'homme du métier.

L'information biologique obtenue en mettant en œuvre le kit QIAamp® DNA stool est quantifiée dans les mêmes conditions que celles permettant de quantifier l'information biologique obtenue en mettant en œuvre le procédé selon la présente invention.

Les résultats obtenus pour chacun des deux protocoles sont présentés dans le tableau 6 infra.

Tableau 6 - Comparaison des résultats obtenus par PCR quantitative à partir de l'ADN extrait par le procédé selon l'invention et par le procédé selon le kit QIAamp® DNA stool (QiaGen).

| Protocole | Quantité d'ADN extrait (μg) | Résultat qPCR (Cq) | | |
|---|---|---|---|---|
| | | MRSA | KPC | *S. pombe* |
| Invention | 2.5-3 | 26.6 | 28.1 | 35.2 |
| QIAamp® DNA stool | 7-10 | 28.4 | 29.2 | Non détecté (>40) |

Ce tableau 6 présente la quantité d'ADN extraite pour chacun des deux protocoles, ainsi que la valeur des Cq obtenus par l'analyse PCR (Cq correspond au Ct ou cycle seuil, le cycle auquel la valeur de fluorescence émise atteint le seuil prédéfini).

Les résultats ainsi obtenus démontrent clairement que l'analyse moléculaire de l'information biologique (ADN) par PCR, effectuée à partir des éluats obtenus par la mise en œuvre du procédé selon l'invention est efficace et présente une sensibilité améliorée par rapport au procédé mettant en œuvre le kit QIAamp® DNA stool. En outre, il est important de noter que seul le procédé selon l'invention permet la détection de levures (ici de levures S. *pombe).*

En conclusion, le procédé d'analyse d'information biologique selon l'invention, mettant en œuvre le dispositif selon l'invention, permet une détection efficace de l'information biologique contenue dans les micro-organismes présents dans un échantillon de selles (y compris concernant les levures), tout en simplifiant les étapes de prélèvement 1), mise en suspension 2) et filtration 3) des échantillons, tel qu'indiqué dans l'exemple 2.

Exemple 6 - Comparaison du procédé d'analyse d'information biologique selon l'invention et du protocole mettant en œuvre le kit QIAamp® DNA stool pour une application de séquençage du microbiote de l'organisme humain.

Le kit QIAamp® DNA stool (QiaGen ; Référence: 51504) est mis en œuvre conformément au protocole expérimental fourni par le fournisseur (QiaGen) et modifié comme mentionné à l'exemple 5 supra.

Les dispositifs objet de la présente invention sont assemblés conformément aux indications données dans l'exemple 1. Le filtrat est collecté conformément à l'exemple 2 et l'ADN est extrait suivant le procédé d'extraction selon l'invention.

Le protocole de traitement du filtrat est identique à celui mentionné dans l'exemple 3 pour du matériel génétique. Toutefois, l'éluat ainsi obtenu est ensuite séquencé par un séquenceur PGM nouvelle génération (IonTorrent) avec une puce 318 (Référence : voir tableau 7 infra).

Tableau 7: Réactifs/kits utilisés pour le séquençage du microbiote

| Réactifs/kits | Stockage | Fournisseur | Réf. | Lot |
|---|---|---|---|---|
| Ion PGM Sequencing Supplies 400 | RT | Life Technologies | 4482003 | 057A03-13 |
| Ion PGM Sequencing Reagents 400 | -20°C | Life Technologies | 4482004 | 057A03-13 |
| Ion PGM Sequencing Solutions 400 | +4°C | Life Technologies | 4482005 | 057A03-13 |
| Ion 318 Chip v2 kit | RT | Life Technologies | 4484354 | P30756-1 |

Afin de tester la reproductibilité du protocole mettant en œuvre la présente invention, trois répétitions par condition expérimentale ont été réalisées pour permettre le calcul de la variabilité de prélèvement (CV), correspondant à l'ensemble des étapes mises en œuvre par le protocole.

Le calcul du CV a été réalisé sur les 10 phylums majoritairement présents dans les selles. Les résultats, présentés dans le tableau 8 ci-dessous, montrent que les CV sont équivalents entre les deux protocoles (10-14%), avec toutefois une forte variation de valeur entre phylum pour le même protocole (6-23% pour le procédé selon l'invention et 2-35% pour QiaGen). Par conséquent, cette expérience montre que le procédé d'analyse d'ADN selon l'invention, permet d'obtenir des résultats équivalents en termes de reproductibilité, tout en étant plus simple à réaliser que le procédé selon le kit QIAamp® DNA stool.

Tableau 8

| CV des 10 phylums majoritairement présents dans les selles | | | | |
|---|---|---|---|---|
| | | | QiaGen | Procédé selon l'invention |
| N° Taxon | Rang | Taxon | CV (%) | CV (%) |
| 1239 | phylum | Firmicutes | 2% | 1% |
| 976 | phylum | Bacteroidetes | 15% | 6% |
| 201174 | phylum | Actinobacteria | 2% | 21% |
| 1224 | phylum | Proteobacteria | 12% | 13% |
| 508458 | phylum | Synergistetes | 6% | 15% |
| 544448 | phylum | Tenericutes | 14% | 23% |
| 203691 | phylum | Spirochaetes | 2% | 17% |
| 1117 | phylum | Cyanobacteria | 35% | 5% |
| 1090 | phylum | Chlorobi | 17% | 23% |
| 3201, 3202 66 | phylum | Fusobacteria | 22% | 17% |
| | | Moyenne | 13% | 14% |
| | | CV (variation des CV) | 82% | 55% |

Exemple 7 - Comparaison en termes de quantification d'ADN d'adénovirus par PCR en utilisant le kit Adenovirus R-gene® (Référence : 69-010B) après extraction d'ADN avec a) le kit d'extraction QIAamp® DNA stool et avec b) le procédé d'extraction d'information biologique selon l'invention.

Afin de contrôler la concentration en adénovirus contenue dans les échantillons testés, des selles contenant une concentration connue en adénovirus (« selle positive ») sont diluées dans un mélange de selles ne contenant pas d'adénovirus. La dilution des selles positives est réalisée en cascade aux concentrations suivantes : $10^4$, $10^5$, $10^6$ et $10^8$ copies de virus par gramme de selle. Dans le cas des selles contenant des adénovirus, les types de Bristol sont généralement compris entre 4 et 7.

Des contrôles sont systématiquement réalisés :

- 2 Contrôles négatifs : Selles non-inoculées détectées négatives et tampon phosphate EDTA non-inoculé.

Pour l'extraction de l'ADN d'adénovirus selon le kit QIAamp® DNA stool (Réf. 51504, QiaGen) le protocole expérimental du fournisseur est suivi.

Le procédé d'extraction d'information biologique (d'ADN d'adénovirus) est décrit ci-après.

Les étapes de 1) prélèvement de selles, 2) mise en suspension et 3) filtration sont effectuées conformément à l'enseignement de l'exemple 2, en utilisant, à titre de solution de mise en suspension, une solution constituée de Phosphate (0.2M), EDTA (50mM), pH8.

Le filtrat ainsi obtenu est vortexé quelques secondes à des fins d'homogénéisation, puis 400 μL de filtrat homogénéisé sont ensuite versés dans le puits d'une navette easyMAG® (bioMérieux) et le programme « Dispense Lysis » est lancé afin de distribuer 2 mL de tampon de lyse (bioMérieux, référence : 280134).

Une fois le programme « Dispense Lysis » terminé, 10 μL d'IC2 (contrôle interne du kit Adénovirus R-gene®), puis 740 μL d'un mélange, contenant 600 μL de tampon de lyse (bioMérieux, référence: 280134) et 140 μL de silice, sont ajouté dans chaque puits de navette easyMAG®. Une fois le mélange réalisé le programme easyMAG® spécifique B, lyse « off-board », élution dans 50 μL est lancé. L'éluat ainsi récupéré est transféré dans un tube neuf dans les 30 minutes, suivant la fin de l'élution.

Une analyse par PCR quantitative est réalisée afin de quantifier l'ADN d'adénovirus extrait suivant le procédé selon l'invention d'une part, et le procédé selon le procédé QIAamp® DNA stool d'autre part, en fonction de la quantité théorique initialement inoculée dans le tampon de mise en suspension. Ainsi, 10 μL d'éluats sont prélevés pour être analysés par PCR quantitative en utilisant le kit Adénovirus R-gene®. Les résultats sont exprimés en log de la concentration en micro-organismes inoculés.

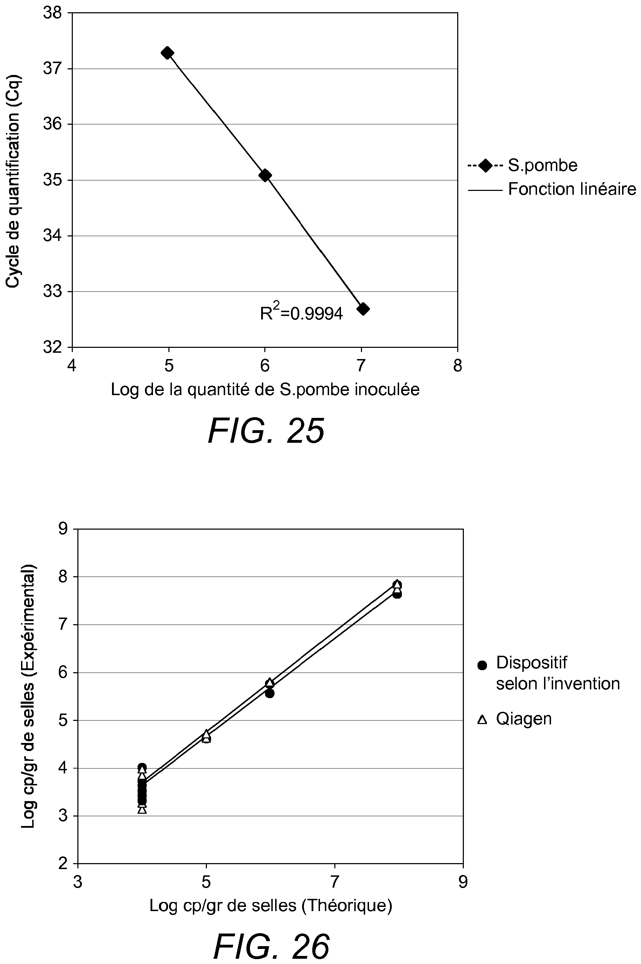

Comme le montrent les résultats présentés en figure 26, l'on note une relation linéaire entre le nombre de virus inoculés préalablement dans le tampon de mise en suspension et la quantité d'ADN détecté en PCR quantitative à partir de l'éluat obtenu en mettant en œuvre le procédé d'extraction d'information biologique selon l'invention. Ainsi, le procédé d'extraction d'information biologique selon l'invention (ADN viral) permet de détecter et quantifier efficacement les adénovirus présents initialement dans un échantillon de selles, et d'obtenir un résultat équivalent au procédé d'extraction mettant en œuvre le kit QIAamp® DNA stool, et ce sur une large gamme de linéarité (de $10^4$ à $10^8$ copie de virus par gramme de selle).

Exemple 8 - Test montrant la quantité d'ADN extrait à partir d'échantillons de selles de type Bristol différents, en mettant en œuvre le procédé d'extraction d'information biologique selon l'invention.

Afin d'évaluer la reproductibilité inter-selles en matière de quantité d'ADN extraite à partir d'échantillons de selles de types de Bristol différents, plusieurs extractions d'acides nucléiques ont été réalisées sur six échantillons de selles différentes, dont le type Bristol est compris entre 1 et 6, en mettant en œuvre le procédé d'extraction d'information biologique (en l'espèce d'ADN) selon l'invention.

Le susdit procédé d'extraction d'information biologique selon l'invention est identique au procédé décrit à l'exemple 4 jusqu'à l'étape faisant intervenir l'easyMAG®. Les éluats obtenus sont analysés au Nanodrop (ThermoScientific), afin de quantifier et de contrôler la pureté (ratio 260/280 et ratio 260/230) de l'ADN extrait.

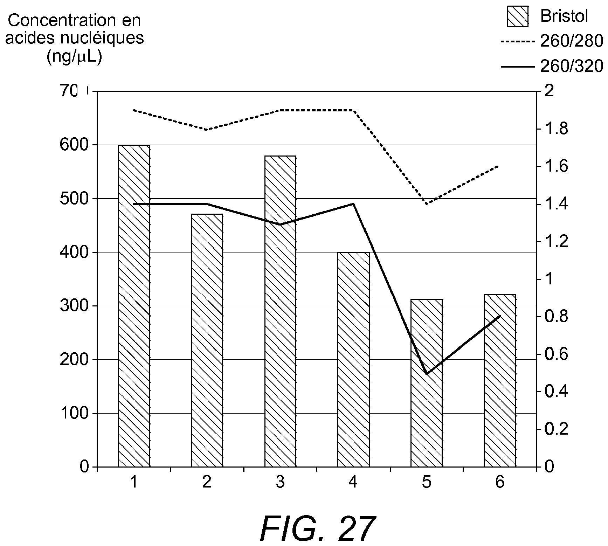

Comme illustré en figure 27, une variation significative de la quantité d'ADN extrait est observée en fonction du type Bristol de l'échantillon de selles. En effet, pour les selles de type Bristol 1 à 3, environ 500 ng/μL d'acides nucléiques (5 μg d'ADN) sont extraits, tandis que pour les selles de type Bristol 4 à 6, la concentration en acides nucléiques se rapproche des 300 ng/μL d'acide nucléique (3 μg d'ADN). Le rendement en masse d'ADN extrait est, par conséquent, environ deux fois moins important pour les selles de type Bristol 4 à 6. Toutefois, la quantité extraite demeure suffisante afin de permettre une analyse par PCR ou par séquençage.

Cette variation en termes de quantité d'ADN extrait n'est pas due à la nature du procédé d'extraction utilisé mais bien au type Bristol des selles. En effet, une extraction d'ADN réalisée à l'aide du kit Macherey Nagel (NucleoSpin Blood L), adapté pour des extractions d'ADN à partir d'échantillons liquides (par exemple à partir de sang) est réalisée sur les selles de type Bristol 5 et 6, et donne des résultats similaires (résultats non représentés). Ce phénomène s'explique par le fait que les selles de type Bristol 5 et 6 sont plus liquides - donc plus diluées par définition - ce qui se traduit par une diminution de la concentration des micro-organismes et donc de facto de leurs ADNs.

En conclusion, le procédé d'extraction d'information biologique (en l'espèce d'ADN) selon l'invention, mettant en œuvre le dispositif selon l'invention, permet d'extraire une quantité suffisante d'ADN de micro-organismes dans des selles de type Bristol 1 à 6 pour permettre une analyse subséquente par PCR ou séquençage, et ce malgré les variations observées sur la figure 27.

A noter que le procédé d'extraction d'information biologique (en l'espèce d'ADN) selon l'invention, mettant en œuvre le dispositif selon l'invention, fonctionne également pour des selles de type Bristol 7 ; l'absence de résultats pour ce type Bristol étant simplement dû à la non-disponibilité de selles de type Bristol 7 au moment où cet exemple a été réalisé.

Exemple 9 - Test démontrant l'efficacité du dispositif selon l'invention pour prélever et analyser des échantillons de selles bovines.

Le prélèvement et l'analyse d'un échantillon de selles bovines sont effectués à l'aide du dispositif dont l'assemblage est décrit à l'exemple 1, à ceci près que :

- le moyen de prélèvement calibré 613 (comprenant trois parties creuses calibrées 6132, 6134 et 6135) du dispositif représenté en figure 16 est utilisé, afin de collecter 3000 mg de selles bovines, et
- le volume du tampon TE est augmenté à 10 mL.

Le dispositif selon l'invention a été utilisé comme indiqué à l'exemple 2 jusqu'à l'étape de filtration incluse.

Le dispositif selon l'invention a ainsi permis d'obtenir 2 mL d'un filtrat de selles bovines, et ce sans colmatage (encombrement) des filtres dudit dispositif.

En conclusion, moyennant quelques ajustements, le dispositif selon l'invention est tout à fait adapté pour une/des application(s) vétérinaire(s), par exemple afin d'obtenir tout ou partie des micro-organismes présents dans un échantillon de selles d'origine animale, par exemple de selles bovines.

Exemple 10 - Comparaison du temps de mise en œuvre et de la praticité d'utilisation entre le procédé d'extraction d'information biologique selon l'invention et le kit QIAamp® DNA stool (QiaGen).

Afin de comparer le temps de mise en œuvre et la praticité d'utilisation entre le procédé d'extraction d'information biologique (en l'espèce d'ADN) selon l'invention et le protocole mettant en œuvre le kit QIAamp® DNA stool, différentes extractions d'ADN ont été effectuées et le temps de réalisation de chaque étape a été reporté dans les tableaux 9, 10 et 11 infra, en fonction du type de procédé utilisé.

Le procédé mettant en œuvre le kit QIAamp® DNA stool a été utilisé pour traiter 5 échantillons de selles. Un total de 25 étapes manuelles (dont 15 de préparation d'échantillons) et une durée totale de 2h20 (dont 1h57 pour la préparation d'échantillons) ont été nécessaires. Les différentes étapes du procédé mettant en œuvre le kit QIAamp® DNA stool et leurs temps de réalisation sont présentés dans le tableau 9 infra :

Tableau 9

| Item n° | Etape du procédé "QIAamp® DNA Stool" | Pour 5 dispositifs | |
|---|---|---|---|
| | | Durée (h:min) | Durée cumulée (h:min) |
| | | | |
| 1 | Préparation réactif et consommable | 00:09 | |
| 2 | Prélèvement de selle 200-250 mg | 00:10 | 00:10 |
| 3 | Préparation du lysozyme | 00:10 | 00:20 |
| 4 | Ajout du lysozyme | 00:06 | 00:26 |
| 5 | Incubation 37°C | 00:30 | 00:56 |
| 6 | Ajout ASL + vortex 15 s/échantillon | 00:05 | 01:01 |
| 7 | Ajout billes + vibroBroyage | 00:08 | 01:09 |
| 8 | Incubation 95°C + Préparation tubes 2mL | 00:10 | 01:19 |
| 9 | Vortex 15s /échantillon. + Centrifugation + Transfert surnageant | 00:06 | 01:25 |
| 10 | Ajout Inhibitex + Vortex + Incubation TA | 00:04 | 01:29 |
| 11 | Centrifugation | 00:06 | 01:35 |
| 12 | Transfert surnageant + Centrifugation | 00:05 | 01:40 |
| 13 | Préparation tubes + PK | 00:01 | 01:41 |
| 14 | Transfert surnageant + Ajout AL + Homogénéisation | 00:04 | 01:45 |
| 15 | Incubation à 70°C | 00:10 | 01:55 |
| 16 | Ajout EtOH + vortex | 00:02 | 01:57 |
| 17 | Dépôt sur la colonne | 00:02 | 01:59 |
| 18 | Centrifugation | 00:02 | 02:01 |
| 19 | Ajout AW1 + Centrifugation | 00:02 | 02:03 |
| 20 | Ajout AW2 + Centrifugation | 00:04 | 02:07 |
| 21 | Ajout AW2 + Centrifugation | 00:05 | 02:12 |
| 22 | Ajout AE | 00:01 | 02:13 |
| 23 | Incubation | 00:05 | 02:18 |
| 24 | Centrifugation | 00:01 | 02:19 |
| 25 | Conservation de l'Eluat | 00:01 | 02:20 |

Par ailleurs, le procédé d'extraction d'information biologique (en l'espèce d'ADN viral et d'ADN bactérien) selon l'invention a également été mis en œuvre pour traiter également 5 échantillons de selles.

Le procédé d'extraction d'ADN viral selon l'invention - pour 5 échantillons - comprend 8 étapes (dont 3 de préparation d'échantillons) et est réalisé en 1h30 à peine (dont 30 minutes de préparation d'échantillons). Les différentes étapes du procédé d'extraction d'ADN viral et leur temps de réalisation sont reportés dans le tableau 10 infra :

Tableau 10

| Item n° | Etape du procédé "ADN Viral" | Pour 5 dispositifs | |
| | | Durée (h:min) | Durée cumulée (h:min) |
| --- | --- | --- | --- |
| | | | |
| 1 | Préparation réactifs et consommables | 00:14 | N.A |
| 2 | Démarrage de l'easyMAG® | 00:13 | N.A |
| 3 | Prélèvement de selles 200-250 mg | 00:04 | 00:04 |
| 4 | Mise en suspension | 00:05 | 00:09 |
| 5 | Filtration | 00:06 | 00:15 |
| 6 | Préparation navette easyMAG® | 00:10 | 00:25 |
| 7 | Lancement protocole easyMAG® | 00:05 | 00:30 |
| 8 | Extraction dans l'easyMAG® | 01:00 | 01:30 |

Le procédé d'extraction d'ADN bactérien - pour 5 échantillons - comprend 12 étapes (dont 7 de préparation d'échantillons) et est réalisé en 2h13 (dont 58 minutes de préparation d'échantillons). Les différentes étapes du procédé d'analyse d'ADN bactérien et leur temps de réalisation sont reportés dans le tableau 11 infra :

Tableau 11

| Item n° | Etape du procédé "ADN Bactérien " | Pour 5 dispositifs | |
| | | Durée (h:min) | Durée cumulée (h:min) |
| --- | --- | --- | --- |
| | | | |
| 1 | Préparation réactifs et consommables | 00:14 | N.A |
| 2 | Démarrage de l'easyMAG® | 00:13 | N.A |
| 3 | Prélèvement de selles 200-250 mg | 00:04 | 00:04 |
| 4 | Mise en suspension | 00:05 | 00:09 |
| 5 | Filtration | 00:06 | 00:15 |
| 6 | Centrifugation | 00:06 | 00:21 |
| 7 | Reprise et Resuspension du culot | 00:12 | 00:33 |
| 8 | Lyse mécanique | 00:21 | 00:54 |
| 9 | Transfert surnageant | 00:04 | 00:58 |
| 10 | Préparation navette easyMAG® | 00:10 | 01:08 |
| 11 | Lancement protocole easyMAG® | 00:05 | 01:13 |
| 12 | Extraction dans l'easyMAG® | 01:00 | 02:13 |

A la lumière des données présentées dans les tableaux 9, 10 et 11 supra, il apparaît que les procédés d'extraction d'ADN bactérien et d'ADN viral selon l'invention, mettant en œuvre le dispositif de l'invention, permettent de limiter le nombre d'étapes et de réduire de manière significative le temps de préparation des échantillons.

A contrario, le procédé mettant en œuvre le kit QIAamp® DNA stool requiert un nombre élevé d'étapes manuelles et techniquement délicates, nécessitant la présence et l'attention continue d'un technicien de laboratoire qualifié.

En conclusion, le dispositif selon l'invention permet de simplifier les protocoles expérimentaux (« ADN viral » et « ADN bactérien ») par rapport aux protocoles de l'art antérieur (QIAamp® DNA stool, QiaGen (Réf. 51504)), en regroupant dans un même dispositif les éléments nécessaires afin d'effectuer les étapes de prélèvement, mise en suspension et filtration. Cette simplification permet de réduire, de manière significative, le nombre et la complexité des manipulations, et de limiter les risques d'erreurs et de contaminations croisées, tout en conférant un confort de travail accru pour le technicien de laboratoire normalement qualifié.

**Bibliographie**

[1] Srijan A, Bodhidatta L, Mason C, Bunyarakyothin G, Jiarakul W, Vithayasai N. Field Evaluation of a Transport Medium and Enrichment Broth for Isolation of Campylobacter Species from Human Diarrheal Stool Samples. J Med Microbiol, 2013; 3, 48-52.

[2] Wasfy M, Oyofo B, Elgindy A, Churilla A. Comparison of préservation media for storage of stool samples. J Clin Microbiol 1995; 33:2176.

**Revendications**

**1.** Dispositif (1) d'obtention de matière biologique et/ou d'information biologique à partir d'un échantillon à matrice hétérogène, par exemple un échantillon de selles humaines ou animales, ledit dispositif comprenant :

- un contenant (10) configuré pour recevoir un contenu comprenant ledit échantillon et au moins une solution de mise en suspension (13), destinée à permettre la mise en suspension dudit échantillon,
- un bouchon (11, 21, 31) permettant de fermer ledit contenant, ledit bouchon comprenant :

- au moins un moyen de prélèvement calibré (113, 213, 313), permettant de prélever un volume prédéterminé dudit échantillon correspondant à une masse donnée, ledit moyen de prélèvement comprenant au moins une partie creuse calibrée (1132, 2132, 3132, 4132, 5132, 6132, 7132) connectée à la partie interne (117) du bouchon et s'étendant de ladite partie interne du bouchon vers l'intérieur du contenant lorsque ledit bouchon et le dit contenant sont assemblés,
- au moins une ouverture (118), permettant la communication de fluide entre l'intérieur dudit contenant et l'extérieur dudit dispositif lorsque ledit bouchon et ledit contenant sont assemblés, au moins un moyen de filtration (116), positionné par rapport à ladite ouverture de façon à filtrer le contenu lors du passage dudit contenu de l'intérieur dudit contenant vers l'extérieur dudit dispositif via ladite ouverture, ledit moyen de filtration étant configuré pour permettre le passage sélectif de ladite matière biologique et/ou information biologique vers l'extérieur dudit dispositif, ledit au moins un moyen de filtration étant sélectionné parmi un filtre à gradient, et une superposition d'au moins deux filtres, dont la taille des pores décroît de l'intérieur vers l'extérieur du dispositif, et **caractérisé en ce que**

ledit contenant comprend en outre au moins un moyen de mise en suspension mécanique, de préférence de forme sphérique telle qu'une bille,
disposé au sein du contenant et configuré pour être mis en mouvement par un ou plusieurs système(s) de force(s) externe(s) au dispositif, par exemple par une agitation manuelle ou induite par l'appareil de malaxage de type vortex, sonificateur ou agitateur magnétique, broyeur à billes,
et **en ce que** ledit moyen de mise en suspension mécanique ayant une taille adaptée pour coopérer avec ladite au moins une partie creuse du moyen de prélèvement calibré, notamment afin de faciliter la mise en suspension de l'échantillon à matrice hétérogène prélevé au préalable.

**2.** Dispositif selon la revendication 1, ledit dispositif comprenant un nombre de moyens de mise en suspension mécanique, tels que des billes, sélectionné entre 1 et 200, de préférence entre 5 et 50, avantageusement entre 10 et 40, de manière particulièrement préférée entre 25 et 35 ; ledit/lesdits moyens de mise en suspension mécanique ayant une taille comprise entre 2 mm et 10 mm, de préférence entre 2,5 mm et 3,5 mm, avantageusement d'environ 3 mm ; de préférence, ledit/lesdits moyens de mise en suspension mécanique étant en verre, fer, plastique(s), céramique(s), de manière particulièrement préférée en verre.

**3.** Dispositif selon l'une des revendications précédentes, dans lequel ledit moyen de prélèvement calibré possède une rigidité suffisante pour éviter que ledit moyen de prélèvement calibré ne se courbe lors du prélèvement d'échantillon.

**4.** Dispositif selon l'une des revendications précédentes, dans lequel ledit contenant comprend ladite solution de mise en suspension, par exemple une solution tampon, dans un volume suffisant pour permettre la mise en suspension de l'échantillon.

**5.** Dispositif selon l'une des revendications précédentes, dans lequel ledit contenant comprend au moins une paroi (101) comportant au moins une zone en matière souple, adaptée pour subir une compression et générer en réponse une surpression à l'intérieur dudit contenant afin de permettre ou faciliter la filtration du contenu au travers dudit moyen de filtration.

**6.** Dispositif selon l'une des revendications précédentes, dans lequel ladite partie creuse calibrée comporte au moins une ouverture permettant de faciliter la mise en suspension dudit échantillon dans ladite solution de mise en suspension.

**7.** Dispositif selon l'une des revendications précédentes, dans lequel ledit bouchon comprend au moins une zone rigide, ladite zone rigide ayant une forme adaptée pour coopérer avec au moins un organe de maintien, tel qu'une pince, connecté à un appareil de malaxage, tel qu'un vortex, de façon à permettre le maintien dudit dispositif sur l'appareil de malaxage lors du malaxage dudit dispositif afin de faciliter la mise en suspension dudit échantillon dans ladite solution de mise en suspension.

**8.** Dispositif selon la revendication 7, ladite zone rigide ayant une forme adaptée pour coopérer avec une pince connectée audit appareil de malaxage, ladite zone rigide comprenant :

- au moins un moyen anti-rotation (220, 3101, 3202), comprenant deux surfaces d'appui distinctes, par exemple constituées par deux épaulements (2201, 2202) ou par deux languettes, chacune des deux surfaces d'appui distinctes étant adaptée pour être ou venir en butée contre une des deux extrémités de la pince lorsque le bouchon subit un mouvement de rotation, afin d'empêcher ou stopper ladite rotation, et/ou
- au moins un moyen anti-translation (221, 321), comprenant au moins une surface d'appui, telle qu'un rebord, une collerette ou un épaulement, ladite au moins une surface d'appui étant adaptée pour être ou venir en butée contre ladite pince lorsque le bouchon subit un mouvement de translation, afin d'empêcher ou stopper ledit mouvement de translation.

**9.** Dispositif selon l'une des revendications précédentes, dans lequel ladite partie creuse calibrée est connectée à la partie interne du bouchon par l'intermédiaire d'une tige (1131, 2131,3131,6131,7131).

**10.** Dispositif selon la revendication précédente, dans laquelle ladite tige comprend ou est connectée à au moins une partie coulissante, permettant de passer d'une première longueur de tige vers une deuxième longueur de tige, ladite deuxième longueur de tige étant inférieure à ladite première longueur de tige ; ladite partie coulissante étant une extension coulissante (8), adaptée pour être positionnée sur ladite tige et coulisser le long de cette dernière afin de passer de ladite première longueur de tige vers ladite deuxième longueur de tige, ladite extension coulissante comprenant au moins une partie creuse calibreé (7131), de préférence positionnée au niveau de l'extrémité de l'extension coulissante la plus éloignée de la partie interne du bouchon.

**11.** Procédé d'obtention de matière biologique et/ou d'information biologique à partir d'un échantillon à matrice hétérogène, ledit procédé mettant en œuvre le dispositif selon l'une des revendications 1 à 10, ledit procédé comprenant les étapes suivantes :

a) prélever un volume d'échantillon prédéterminé correspondant à une masse donnée à l'aide dudit moyen de prélèvement calibré (171), mettre en suspension ledit échantillon dans ladite solution de mise en suspension, éventuellement par mise en suspension mécanique (172), filtrer (173) la suspension obtenue à l'étape b) à travers ledit moyen de filtration afin d'obtenir un filtrat contenant ladite matière biologique et/ou ladite information biologique.

**12.** Procédé d'obtention selon la revendication 11, configuré pour permettre l'obtention de matière biologique et/ou d'information biologique à partir d'un échantillon à matrice hétérogène, de préférence pour l'obtention de matière biologique, préférablement pour l'obtention de matière biologique microscopique, avantageusement pour l'obtention de matière microbiologique.

**13.** Procédé d'obtention selon la revendication 12, dans lequel l'échantillon à matrice hétérogène est sélectionné parmi un échantillon de sol, un échantillon de selles, un échantillon médico-légal tel qu'un échantillon de tissu nécrosé, un échantillon alimentaire, un échantillon industriel, de préférence ledit échantillon étant un échantillon de selles humaines ou animales.

14. Procédé d'extraction d'information biologique à partir d'un échantillon à matrice hétérogène, ledit procédé comprenant les étapes suivantes :

a) mettre en œuvre le procédé selon la revendication 11 afin d'obtenir un filtrat contenant ladite matière biologique et/ou ladite information biologique,
b) lorsque l'information biologique devant être extraite est contenue dans ladite matière biologique, telle qu'une cellule, une bactérie, un champignon ou une levure, réaliser une étape de lyse de ladite matière biologique, de préférence une étape de lyse mécanique, afin d'obtenir un lysat comprenant l'information biologique devant être extraite, c) extraire l'information biologique à partir du filtrat obtenu à l'étape a) ou du lysat obtenu à l'étape b) en mettant en œuvre un procédé d'extraction d'information biologique adapté.

15. Procédé d'extraction d'information biologique selon la revendication précédente, ledit procédé comprenant avant l'étape c), et avant l'étape b) lorsque celle-ci doit être effectuée, une étape de concentration (1741) de ladite matière biologique et/ou de ladite information biologique, de préférence par centrifugation ou par floculation.

16. Procédé d'analyse d'information biologique, ledit procédé comprenant les étapes suivantes :

a) extraire l'information biologique à analyser en mettant en œuvre le procédé selon la revendication 14 ou 15,
b) identifier et/ou quantifier (17450) ladite information biologique par toute méthode d'analyse

appropriée, par exemple une méthode d'analyse génétique telle qu'une PCR, une méthode de type immunodosage ou un essai enzymatique.

17. Procédé d'analyse de matière biologique à partir d'un échantillon à matrice hétérogène, ledit procédé comprenant les étapes suivantes :

a) obtenir un filtrat contenant la matière biologique à analyser en utilisant le procédé selon la revendication 11,
b) le cas échéant, inoculer un milieu réactionnel avec le filtrat obtenu à l'étape a), ledit milieu réactionnel étant adapté pour permettre la croissance et/ou l'expression d'au moins un métabolisme de ladite matière biologique,
c) le cas échant, incuber le filtrat obtenu à l'étape a), ou le milieu réactionnel inoculé obtenu à l'étape b), durant un laps de temps et à une température appropriés,
d) analyser la matière biologique à l'issue de l'étape a), b) ou c) par tout moyen d'analyse biologique approprié.

18. Procédé d'analyse selon la revendication 17, ledit procédé comprenant l'étape b) et éventuellement l'étape c), avantageusement les étapes b) et c), dans lequel :

- l'étape c), lorsqu'elle est présente, consiste à incuber le milieu réactionnel inoculé obtenu à l'étape b) durant un laps de temps et à une température appropriés, et
- l'étape d'analyse de matière biologique d) consiste en la détection et/ou l'identification et/ou le dénombrement de ladite matière biologique sur/dans ledit milieu réactionnel, de préférence par lecture visuelle ou optique.

19. Kit d'obtention de matière biologique et/ou d'information biologique à partir d'un échantillon à matrice hétérogène, ledit kit comprenant le dispositif selon l'une quelconque des revendications 1 à 10 et au moins une solution de mise en suspension, configurée pour permettre la mise en suspension dudit échantillon.

**Patentansprüche**

1. Vorrichtung (1) zur Gewinnung von biologischem Material und/oder biologischer Information aus einer Probe mit heterogener Matrix, beispielsweise einer Probe von menschlichem oder tierischem Stuhl, wobei die Vorrichtung Folgendes umfasst:

- einen Behälter (10), der konfiguriert ist, um einen Inhalt aufzunehmen, der die Probe und zumindest eine Suspensionslösung (13), die dazu bestimmt ist, die Probe zu suspendieren, umfasst,
- einen Verschluss (11, 21, 31), der es ermöglicht, den Behälter zu verschließen, wobei der Verschluss Folgendes umfasst:

- zumindest ein kalibriertes Probenentnahmemittel (113, 213, 313), das es ermöglicht, ein vorbestimmtes Volumen der Probe, das einer gegebenen Masse entspricht, wobei das Entnahmemittel zumindest einen kalibrierten hohlen Teil (1132, 2132, 3132, 4132, 5132, 6132, 7132) umfasst, der mit dem inneren Teil (117) des Verschlusses verbunden ist und sich vom inneren Teil des Verschlusses in das Innere des Behälters erstreckt, wenn der Verschluss und der Behälter zusammengesetzt sind,
- zumindest eine Öffnung (118), die eine Fluidverbindung zwischen dem Inneren des Behälters und dem Äußeren der Vorrichtung ermöglicht, wenn der Verschluss und der Behälter zusammengesetzt sind,
- zumindest ein Filtermittel (116), das in Bezug auf die Öffnung so positioniert ist, dass es den Inhalt beim Durchgang des Inhalts vom Inneren des Behälters über die Öffnung zum Äußeren der Vorrichtung filtert, wobei das Filtermittel konfiguriert ist, um den selektiven Durchgang des biologischen Materials und/oder der biologischen Information zum Äußeren der Vorrichtung zu ermöglichen, wobei das zumindest eine Filtermittel ausgewählt ist aus einem Gradientenfilter und einer Überlagerung von mindestens zwei Filtern, deren Porengröße vom Inneren zum Äußeren der Vorrichtung hin abnimmt, und

**dadurch gekennzeichnet, dass** der Behälter ferner zumindest ein mechanisches Suspensionsmittel umfasst, das vorzugsweise eine sphärische Form wie eine Kugel aufweist, innerhalb des Behälters angeordnet und konfiguriert ist, um durch ein oder mehrere außerhalb der Vorrichtung befindliche(s) Kraftsystem(e) in Bewegung gesetzt zu werden, beispielsweise durch manuelles Rühren oder Rühren, das durch das Mischgerät vom Typ eines Vortexmischers, Sonifikators oder Magnetrührers oder einer Kugelmühle induziert wird, und dass das mechanische Suspensionsmittel eine Größe aufweist, die geeignet ist, um mit dem zumindest einen hohlen Teil des kalibrierten Probenentnahmemittels zusammenzuwirken, insbesondere um das Suspendieren der zuvor entnommenen Probe mit heterogener Matrix zu erleichtern.

2. Vorrichtung nach Anspruch 1, wobei die Vorrichtung eine Anzahl von mechanischen Suspensionsmitteln, beispielsweise Kugeln, aufweist, die zwischen 1 und 200, vorzugsweise zwischen 5 und 50, vorteilhafterweise zwischen 10 und 40, besonderes vorteilhafterweise zwischen 25 und 35, liegt; wobei das/die mechanische/-n Suspensionsmittel eine Größe zwischen 2 mm und 10 mm, vorzugsweise zwischen 2,5 mm und 3,5 mm, vorteilhafterweise etwa 3 mm, aufweisen; wobei das/die mechanische/-n Suspensionsmittel aus Glas, Eisen, Kunststoff(en), keramischem/-n Stoff/-en, besonders vorzugsweise aus Glas, sind.

3. Vorrichtung nach einem der vorangehenden Ansprüche, wobei das kalibrierte Probenentnahmemittel eine ausreichende Steifigkeit besitzt, um zu verhindern, dass sich das kalibrierte Probenentnahmemittel während der Probenentnahme verbiegt.

4. Vorrichtung nach einem der vorangehenden Ansprüche, wobei der Behälter die Suspensionslösung, beispielsweise eine Pufferlösung, in einem Volumen umfasst, das ausreichend ist, um das Suspendieren der Probe zu ermöglichen.

5. Vorrichtung nach einem der vorangehenden Ansprüche, wobei der Behälter zumindest eine Wand (101) umfasst, die zumindest einen Bereich aus weichem Material aufweist, der geeignet ist, um eine Kompression zu erfahren und in Reaktion darauf einen Überdruck im Inneren des Behälters zu erzeugen, um die Filtration des Inhalts durch das Filtermittel zu ermöglichen oder zu erleichtern.

6. Vorrichtung nach einem der vorangehenden Ansprüche, wobei der kalibrierte hohle Teil zumindest eine Öffnung aufweist, die es ermöglicht, das Suspendieren der Probe in der Suspensionslösung zu erleichtern.

7. Vorrichtung nach einem der vorangehenden Ansprüche, wobei der Verschluss zumindest einen starren Bereich umfasst, wobei der starre Bereich eine Form aufweist, die geeignet ist, mit zumindest einem Halteelement, beispielsweise einer Klemme, zusammenzuwirken, das mit einem Mischgerät, beispielsweise einem Vortexmischer, verbunden ist, um zu ermöglichen, dass die Vorrichtung während des Mischvorgangs der Vorrichtung am Mischgerät gehalten wird, um das Suspendieren der Probe in der Suspensionslösung zu erleichtern.

8. Vorrichtung nach Anspruch 7, wobei der starre Bereich eine Form aufweist, die geeignet ist, um mit einer mit dem Mischgerät verbundenen Klemme zusammenzuwirken, wobei der starre Bereich Folgendes umfasst:

- zumindest ein Antirotationsmittel (220, 3101, 3202), das zwei unterschiedliche Auflageflächen umfasst, die beispielsweise durch zwei Schultern (2201, 2202) oder durch zwei Laschen gebildet sind, wobei jede der beiden unterschiedlichen Auflageflächen dazu geeignet ist, an einem der beiden Enden der Klemme anzuliegen oder zur Anlage zu kommen, wenn der Verschluss eine Drehbewegung erfährt, um die Drehung zu verhindern oder zu stoppen, und/oder

- zumindest ein Antitranslationsmittel (221, 321), das zumindest eine Auflagefläche, beispielsweise einen Flansch, einen Kragen oder eine Schulter, umfasst, wobei die zumindest eine Auflagefläche geeignet ist, an der Klemme anzuliegen oder zur Anlage zu kommen, wenn der Verschluss eine Translationsbewegung erfährt, um die Translationsbewegung zu verhindern oder zu stoppen.

9. Vorrichtung nach einem der vorangehenden Ansprüche, wobei der kalibrierte hohle Teil über eine Stange (1131, 2131, 3131, 6131, 7131) mit dem inneren Teil des Verschlusses verbunden ist.

10. Vorrichtung nach dem vorangehenden Anspruch, wobei die Stange zumindest einen gleitenden Teil umfasst oder mit diesem verbunden ist, der den Übergang von einer ersten Stangenlänge zu einer zweiten Stangenlänge ermöglicht, wobei die zweite Stangenlänge kleiner als die erste Stangenlänge ist; wobei der gleitende Teil eine gleitende Verlängerung (8) ist, die geeignet ist, auf der Stange positioniert zu sein und entlang derselben zu gleiten, um von der ersten Stangenlänge zur zweiten Stangenlänge überzugehen, wobei die gleitende Verlängerung zumindest einen kalibrierten hohlen Teil (7131) umfasst, der vorzugsweise an dem Ende der gleitenden Verlängerung positioniert ist, das am weitesten vom inneren Teil des Verschlusses entfernt ist.

11. Verfahren zur Gewinnung von biologischem Material und/oder biologischer Information aus einer Probe mit heterogener Matrix, wobei das Verfahren die Vorrichtung nach einem der Ansprüche 1 bis 10 implementiert, wobei das Verfahren die folgenden Schritte umfasst:

a) Entnehmen eines vorbestimmten Probenvolumens, das einer gegebenen Masse entspricht, mit Hilfe des kalibrierten Probenentnahmemittels (171),
b) Suspendieren der Probe in der Suspensionslösung, eventuell durch mechanisches Suspendieren (172), Filtern (173) der in Schritt
c) erhaltenen Suspension durch das Filtermittel, um ein Filtrat zu erhalten, das das biologische Material und/oder die biologische Information enthält.

12. Gewinnungsverfahren nach Anspruch 11, das konfiguriert ist, um die Gewinnung von biologischem Material und/oder biologischer Information aus einer Probe mit heterogener Matrix zu ermöglichen, vorzugsweise zur Gewinnung von biologischem Material, vorzugsweise zur Gewinnung von mikroskopischem biologischem Material, vorteilhafterweise zur Gewinnung von mikrobiologischem Material.

13. Gewinnungsverfahren nach Anspruch 12, wobei die Probe mit heterogener Matrix aus einer Bodenprobe, einer Stuhlprobe, einer forensischen Probe, beispielsweise eines nekrotischen Gewebes, einer Lebensmittelprobe und einer industriellen Probe ausgewählt ist, wobei die Probe vorzugsweise eine menschliche oder tierische Stuhlprobe ist.

14. Verfahren zur Extraktion von biologischer Information aus einer Probe mit heterogener Matrix, wobei das Verfahren die folgenden Schritte umfasst:

a) Durchführen des Verfahrens nach Anspruch 11, um ein Filtrat zu erhalten, das das biologische Material und/oder die biologische Information enthält,
b) wenn die zu extrahierende biologische Information im biologischen Material, beispielsweise einer Zelle, einem Bakterium, einem Pilz oder einer Hefe, enthalten ist, Ausführen eines Lyseschritts des biologischen Materials, vorzugsweise eines mechanischen Lyseschritts, um ein Lysat zu erhalten, das die zu extrahierende biologische Information enthält,
c) Extrahieren von biologischer Information aus dem in Schritt a) erhaltenen Filtrat oder aus dem in Schritt b) erhaltenen Lysat durch Ausführen eines geeigneten Verfahrens zur Extraktion von biologischer Information.

15. Verfahren zur Extraktion von biologischer Information nach dem vorangehenden Anspruch, wobei das Verfahren vor dem Schritt c) und vor dem Schritt b), sofern dieser durchgeführt werden soll, einen Schritt des Konzentrierens (1741) des biologischen Materials und/oder der biologischen Information umfasst, vorzugsweise durch Zentrifugieren oder durch Ausflocken.

16. Verfahren zur Analyse von biologischer Information, wobei das Verfahren die folgenden Schritte umfasst:

a) Extrahieren von zu analysierender biologischer Information durch Ausführen des Verfahrens nach Anspruch 14 oder 15,

b) Identifizieren und/oder Quantifizieren (17450) der biologischen Information durch ein beliebiges geeignetes Analyseverfahren, beispielsweise ein genetisches Analyseverfahren wie eine PCR, ein Immunoassay-Verfahren oder einen Enzymassay.

**17.** Verfahren zur Analyse von biologischem Material aus einer Probe mit heterogener Matrix, wobei das Verfahren die folgenden Schritte umfasst:

a) Gewinnen eines Filtrats, das das zu analysierende biologische Material enthält, anhand des Verfahrens nach Anspruch 11,

b) gegebenenfalls Inokulieren eines Reaktionsmediums mit dem in Schritt a) gewonnenen Filtrat, wobei das Reaktionsmedium geeignet ist, das Wachstum und/oder den Ausdruck eines Metabolismus des biologischen Materials zu ermöglichen,

c) gegebenenfalls Inkubieren des in Schritt a) erhaltenen Filtrats oder des in Schritt b) erhaltenen inokulierten Reaktionsmediums über einen geeigneten Zeitraum und bei einer geeigneten Temperatur,

d) Analysieren des biologischen Materials nach Schritt a), b) oder c) durch ein beliebiges geeignetes biologisches Analysemittel.

**18.** Analyseverfahren nach Anspruch 17, wobei das Verfahren Schritt b) und eventuell Schritt c), vorteilhafterweise Schritt b) und c), umfasst, wobei:

- Schritt c), sofern vorhanden, darin besteht, das in Schritt b) erhaltene inokulierte Reaktionsmedium über einen geeigneten Zeitraum und bei einer geeigneten Temperatur zu inkubieren, und

- Schritt d) zur Analyse von biologischem Material in der Erkennung und/oder der Identifizierung und/oder der Auszählung des biologischen Materials auf/in dem Reaktionsmedium besteht, vorzugsweise durch visuelles oder optisches Auslesen.

**19.** Kit zur Gewinnung von biologischem Material und/oder biologischer Information aus einer Probe mit heterogener Matrix, wobei das Kit die Vorrichtung nach einem der Ansprüche 1 bis 10 und zumindest eine Suspensionslösung umfasst, die konfiguriert ist, um das Suspendieren der Probe zu ermöglichen.

**Claims**

**1.** A device (1) for obtaining biological material or biological information from a sample with a heterogeneous matrix, for example a sample of human or animal stools, the device comprising:

- a container (10) configured to receive a content comprising the sample and at least one suspending solution (13) intended to allow suspending said sample,
- a cap (11, 21, 31) configured to close the container, the cap comprising:

  ■ at least one calibrated sampling element to take a predetermined volume of said sample corresponding to a given mass; said calibrated sampling element (113, 213, 313) comprising at least one calibrated hollow part (1132, 2132, 3132, 4132, 5132, 6132, 7132) connected to an internal part (117) of the cap and extending from the internal part of the cap to the interior of the container when said cap and container are assembled together,
  ■ at least one opening (118) allowing the fluid communication between the interior of the container to the exterior of the device when the cap and container are assembled together,
  ■ at least one filtration element (116) arranged to filter the content when said content passes from the interior of the container to the exterior of the device via the at least one opening; said filtration element being configured to allow the selective passage of said biological material and/or biological information out of said device, said at least one filtration means being selected from among a gradient filter, and a superposition of at least two filters, the pore size of which decreases from the inside to the outside of the device,

**characterized in that** the container further comprises at least one mechanical suspension element, preferably of spherical shape such as a ball, disposed within the container and configured to be moved by one or more force system external to the device, for example by manual stirring or induced by the vortex, sonicator or magnetic stirrer, bead beater and

**in that** the at least one mechanical suspension element has a size adapted to cooperate with the at least one calibrated hollow part of the calibrated sampling element, preferably in order to facilitate the suspension of the sample with a heterogeneous matrix previously sampled.

2. Device according to claim 1, comprising between 1 and 200 mechanical suspension element, such as beads, and preferably between 5 and 50 and advantageously between 10 and 40 and more preferably between 25 and 35; the mechanical suspension element(s) having a diameter of between 2mm and 10 mm, preferably between 2,5 mm and 3,5 mm, and advantageously around 3 mm; preferably the mechanical suspension element(s) are made from a material selected from the group consisting of glass, iron, plastics, and ceramics and more preferably from glass.

3. Device according to one of the preceding claims, wherein the calibrated sampling element has a rigidity sufficient to avoid that the calibrated sampling element bends during sampling of the sample.

4. Device according to one of the preceding claims, wherein the container comprises the suspending solution, being for example a buffer, in a volume sufficient to suspend the sample.

5. Device according to one of the preceding claims, wherein the container further comprises at least one wall (101), the wall comprising at least one flexible area suitable for undergoing compression to generate overpressure inside of the container to allow or to facilitate filtration of the content through the filtration element.

6. Device according to one of the preceding claims, wherein the calibrated hollow part comprises at least one opening allowing facilitating suspension of the sample in the suspending solution.

7. Device according to one of the preceding claims, wherein the cap further comprises at least one rigid area having a shape adapted for cooperating with at least one maintaining member, such as a clip, connected to a mixing apparatus, such as a vortex, to allow the device to be maintained on the mixing apparatus during mixing to facilitate the suspension of the sample in the suspending solution.

8. Device according to claim 7, wherein the rigid area has a form adapted for cooperating with a clip connected to the mixing apparatus, said rigid area comprising :

   - at least one anti-rotation element (220, 3101, 3202) comprising two bearing surfaces distinct from each other, for example constituted by two shoulders (2201, 2202) or two tabs, each of the distinct bearing surfaces being configured to be or to come against one of the two extremities of the clip when the cap rotates, in order to prevent or stop said rotation,
   - at least one anti-translation element (221, 321) comprising at least one bearing surface such as a lip, a collar or a shoulder, said at least one bearing surface being configured to be or to come against the clip when the cap translates, in order to prevent or stop the translation.

9. Device according to one of the preceding claims, wherein the calibrated hollow part is connected to the internal part of the cap with a rod (1131, 2131, 3131, 6131, 7131).

10. Device according to the preceding claim, wherein the rod further comprises or is connected to at least one sliding part allowing to slide from a first length of the rod to a second length of the rod, said second length of the rod being smaller than the first length of the rod, the sliding part being a sliding extension (8) configured to be arranged on the rod and to slide along said rod from the first length of the rod to the second length of the rod, said sliding extension comprising at least the calibrated hollow part (7131), preferably arranged at the vicinity of the end of the sliding extension furthest from the internal part of the cap.

11. A method for obtaining biological material and/or biological information from a sample with a heterogeneous matrix using a device according to any of the claims 1 to 10, said method comprising:

   a) obtaining a predetermined volume of a sample corresponding to a given mass using a calibrated sampling element (171);
   b) suspending the sample in a suspending solution; eventually by mechanical suspension (172) and
   c) filtering (173) the suspension obtained in step b) through a filtration element to obtain a filtrate containing the biological material or biological information,

**12.** Method according to claim 11, configured to allow obtaining of biological material and/or information from a sample with heterogeneous matrix, preferably for obtaining a biological material, preferentially for obtaining microscopic biological material and advantageously for obtaining microbiological material.

**13.** Method according to claim 12, wherein the sample with heterogeneous matrix is selected from a soil sample, a human stool sample, an animal stool sample, a medico-legal sample, a food sample and an industrial sample and preferably from a human or animal stool sample.

**14.** Method for extracting biological information from a sample with heterogeneous matrix, said extraction method comprising the following steps :

a) Implementing the method according to claim 11 in order to obtain a filtrate containing a biological material and/or a biological information,
b) lysing the biological material preferably by mechanical lysis, in order to obtain a lysate comprising the biological information intended to be extracted, when the biological information intended to be extracted is contained in the biological material, such as a cell, a bacterium, a mold or a yeast,
c) extracting the biological information from the filtrate in step a) or from the lysate in step b) by implementing a method adapted for extracting the biological information.

**15.** Method for extracting biological information according to the preceding claim, comprising before step c) and before step b) when step b) must be done, a step of concentration (1741) of biological material and/or biological information, preferably by centrifugation or by flocculation.

**16.** A method for analyzing biological information comprising the following steps :

a) extracting the biological information to be analyzed with the method according claim 14 or 15, and
b) identifying or quantifying (17450) the biological information by any appropriate method of analysis, for example by an analysis method such as a PCR, or a method of immunodosage ou an enzymatic assay.

**17.** A method for analyzing biological material from a sample with heterogeneous matrix, said method comprising the following steps :

a) Obtaining a filtrate containing the biological material to be analyzed by using the method according to claim 11,
b) If applicable, Inoculating a reactional medium with the filtrate obtained in step a), said reactional medium being adapted to allow the growth and/or the expression of at least one metabolism of biological material,
c) If applicable, Incubating the filtrate obtained in step a) or the reactional medium inoculated in step b) during an appropriate period at an appropriate temperature,
d) Analyzing the biological material following step a), b), or c) by any biological analyzing means which is appropriate.

**18.** Method according to claim 17, comprising step b) and eventually step c) and advantageously steps b) and c), wherein :
e) step c), when done, consists in incubating the reactional medium inoculated obtained in step b) during an appropriate period at an appropriate temperature and

- the analysis step of biological material d) consists in detecting and/or quantifying and/or identifying said biological material on or in the reactional medium and preferably by visual or optical reading.

**19.** A kit for obtaining biological material and/or biological information from a sample with a heterogeneous matrix, the kit comprising the device according to any of the claims 1 to 10 and at least one suspending solution configured to allow the suspension of said sample.

FIG. 1B

FIG. 1A

FIG. 2

FIG. 3

**FIG. 4**

**FIG. 5**

EP 3 185 782 B1

FIG. 6

FIG. 7

FIG. 8A

FIG. 8B

**FIG. 9**

**FIG. 10**

FIG. 11

FIG. 12

*FIG. 13A*

*FIG. 13B*

FIG. 14

FIG. 15

FIG. 16

EP 3 185 782 B1

FIG. 17    FIG. 18    FIG. 19    FIG. 20

FIG. 21

Prélèvement calibré d'un échantillon à matrice hétérogène ⌇~171

↓

Mise en suspension mécanique ⌇~172

↓

Stockage ⌇~1721

↓

Filtration de la suspension / Isolement de matière biologique et/ou d'information biologique ⌇~173

Analyse de l'information biologique

Analyse de matière biologique

concentration/enrichissement de matière biologique ⌇~1750

1741~ Concentration de matière biologique et/ou d'information biologique

Analyse Microbienne/Virale

Technique d'analyse de matière biologique viable ⌇~1751

1742~ Lyse pour libérer l'information biologique

Analyse Microbienne

Détection/Identification/ Dénombrement de la matière biologique ⌇~1752

Analyse génétique

Analyse protéique / de métabolite

17431~ Extraction des acides nucléiques

Technique d'analyse protéique /de métabolite ⌇~17441

17432~ Techniques d'analyse génétique

Détection/Identification/Quantification de l'information biologique ⌇~17450

## FIG. 22

*FIG. 23*

*FIG. 24*

FIG. 25

FIG. 26

FIG. 27

## RÉFÉRENCES CITÉES DANS LA DESCRIPTION

**Documents brevets cités dans la description**

- US 4081356 A
- US 7648681 B

**Littérature non-brevet citée dans la description**

- **SRIJAN A ; BODHIDATTA L ; MASON C ; BUN-YARAKYOTHIN G ; JIARAKUL W ; VITHAYASAI N.** Field Evaluation of a Transport Medium and Enrichment Broth for Isolation of Campylobacter Species from Human Diarrheal Stool Samples. *J Med Microbiol,* 2013, vol. 3, 48-52

- **WASFY M ; OYOFO B ; ELGINDY A ; CHURILLA A.** Comparison of préservation media for storage of stool samples. *J Clin Microbiol,* 1995, vol. 33, 2176